# EUROPEAN PATENT SPECIFICATION

(11) **EP 3 139 969 B1**
(45) Date of publication and mention of the grant of the patent: **11.07.2018**
(21) Application number: 15725918.5
(22) Date of filing: 04.05.2015
(51) Int. Cl.: A61L 15/22, A61L 15/60

(54) **HOMOGENOUS FILM COMPOSITIONS**
HOMOGENE FILMZUSAMMENSETZUNGEN
COMPOSITIONS DE FILM HOMOGÈNE

(30) Priority: 05.05.2014 US 201461988379 P
(43) Date of publication of application: 15.03.2017
(73) Proprietor: Lubrizol Advanced Materials, Inc., Cleveland, OH 44141-3247 (US)
(72) Inventor: MIINEA, Liliana A., Cleveland, Ohio 44141-3247 (US); MARCHANT, Nancy S., Cleveland, Ohio 44141-3247 (US); BAXTER, James R., Cleveland, Ohio 44141-3247 (US); ROHR, Eric L., Cleveland, Ohio 44141-3247 (US); CAMARDO, Andrew, Cleveland, Ohio 44141-3247 (US)
(74) Representative: dompatent von Kreisler Selting Werner - Partnerschaft von Patent- und Rechtsanwälten mbB
(86) International application number: PCT/US2015/029009
(87) International publication number: WO 2015/171483

(56) References cited:
- EP-A1- 1 829 563
- WO-A1-2013/180937

## Description

The invention relates to a homogenous film composition containing a poly(acrylic) acid polymer and a thermoplastic polyurethane composition, as defined by claim 1. The film provides fluid absorption properties associated with the poly(acrylic) acid polymer while also exhibiting the mechanical properties typically derived from thermoplastic polyurethanes. The films provide useful materials for medical and pharmaceutical applications.

### BACKGROUND OF THE INVENTION

Conventional wound treatment typically involves covering the wound with a primary dressing to prevent further contamination and infection, to retain moisture, and to absorb wound exudate. Wound exudate is a generic term used to describe the liquid produced from chronic wounds, fistulae or acute wounds once hemostasis has been achieved. As excessive exudate can cause maceration of the surrounding skin or surface of a wound, which in turn can lead to infection, considerable attention has been given to the development of wound dressings that prevent the accumulation of large volumes of fluid within a wound and spreading of the fluid over surrounding healthy tissue. Document WO2013 180937 A1 shows an absorbent article comprising a layer made of a mixture of poly(acrylic)acid and polyurethane. The two polymers do not form a (semi)interpenetrating network.

Despite the large number of wound dressings available, there is still a need for improved, "intelligent" dressings that are capable of adjusting their properties according to the progression of wound healing, have an antimicrobial effect, reduce odor and stimulate cell function and the healing process, among others.

### SUMMARY OF THE INVENTION

The disclosed technology provides a composition that displays film-forming properties and fluid absorption characteristics. The film as disclosed herein provides the fluid absorption properties associated with a poly(acrylic) acid polymer in combination with good mechanical properties attributed to a thermoplastic polyurethane (TPU).

The invention provides a homogenous film including a partially-neutralized, cross-linked poly(acrylic) acid polymer; and a hydrophilic thermoplastic polyurethane, as defined by claim 1.

The invention further provides the homogenous film described herein in which the cross-linked poly(acrylic) acid is a carbomer copolymer, a carbopol homopolymer, a carbopol interpolymer or polycarbophil.

The invention further provides the homogenous film described herein in which the poly(acrylic) acid polymer is cross-linked with an allyl ether cross-linking agent.

The invention further provides the homogenous film described herein in which the allyl ether cross-linking agent includes one or more of an allyl pentaerythritol, allyl sucrose, trimethylolpropane diallyl ether (TMPDE) and divinyl glycol.

The invention further provides the homogenous film described herein in which the thermoplastic polyurethane includes the reaction product of (i) a polyisocyanate component including at least on aliphatic diisocyanate; (ii) a polyol component including at least one polyether polyol; and (iii) a chain extender component.

The invention further provides the homogenous film described herein in which the chain extender component comprises an aliphatic diol.

The invention further provides the homogenous film described herein in which the polyol component includes at least one polyethylene glycol having a number average molecular weight (Mn) of at least 300.

The invention further provides the homogenous film described herein in which the polyol component includes at least one polyethylene glycol having a number average molecular weight (Mn) of at least 1450.

The invention further provides the homogenous film described herein in which the polyol component includes a blend of polyethylene glycol having number average molecular weights (Mn) of at least 1450 and at least 8000.

The invention further provides the homogenous film described herein in which the cross-linked poly(acrylic) acid polymer is partially neutralized.

The invention further provides the homogenous film described herein in which the water absorption of the film is from about 400% to about 3000% by weight of dry film.

The invention further provides the homogenous film described herein in which the ratio of the partially-neutralized, cross-linked poly(acrylic) acid polymer to hydrophilic thermoplastic polyurethane is from 1:1 to 1:60.

The invention further provides the homogenous film described herein in which the hydrophilic thermoplastic polyurethane forms about 97-50% of the total weight of the composition.

The invention further provides the homogenous film described further including a therapeutically active agent dispersed therein.

The invention further provides a wound dressing including the homogenous film described herein.

The invention further provides a wound dressing in which the homogenous film described herein includes a single layer film, as defined by claim 14.

The invention further provides a wound dressing in which the single layer of the homogenous film described herein is from 0.5 to 100 mil thick.

The invention further provides a patch including the homogenous film described herein, as defined by claim 17.

The invention further provides a patch further including one or more of a pharmaceutical, a biologically active compound, an absorptive material, a personal care compound, an active ingredient, a therapeutic aid, or combinations thereof.

The invention further provides a patch in which the patch is an adhesive patch or a non-adhesive patch.

The invention further provides a film having, upon drying, a tensile strength of from 5 MPa to 50 MPa as measured by ASTM D882-12; a percent elongation from 100 to 700 as measured by ASTM D882-12; and a Young's modulus from 3 MPa to 150 MPa as measured by ASTM D882-12.

The invention further provides a film, having, upon drying, a MVTR of from 1000 to 8000 g/(m²xday).

### BRIEF DESCRIPTION OF THE DRAWING FIGURES

Fig. 1 is a graphical representation illustrating the fluid absorption of films prepared according to the present invention and exemplified in Examples 1-4 versus a solvent cast film of commercial thermoplastic polyurethane film.
Fig. 2 is a graphical representation illustrating percent elongation (horizontal) of films according to the present invention and exemplified in Examples 1-4 versus comparative Example 1-2.
Fig. 3 is a graphical representation illustrating the percent elongation of films according to the present invention and exemplified in Examples 1-4 versus commercially available wound dressing films.
Fig. 4 is a graphical representation illustrating the moisture vapor transmission rate of films according to the present invention and exemplified in Examples 1-4 versus comparative Examples 1-2.
Fig. 5 is a graphical representation illustrating the moisture vapor transmission rate of films according to the present invention and exemplified in Examples 1-4 versus commercially available wound dressing films.

### DETAILED DESCRIPTION OF THE INVENTION

Various preferred features and embodiments will be described below by way of non-limiting illustration.

The homogenous film described herein is prepared from a gel-like solution containing at least two polymers, namely, a partially-neutralized, cross-linked poly(acrylic) acid polymer and a hydrophilic thermoplastic polyurethane (TPU). By "gel-like" it is meant that the viscosity will, in one embodiment, be from 600 Cps to 50,000 Cps, and in another embodiment, from 3,000 to 35, 000 Cps, or from 3,000 to 15,000 Cps, as tested by Brookfield rotating spindle method. By homogenous it is meant that the film is present as a single phase with a uniform appearance and composition throughout. The two polymers, upon mixing will form an interpenetrating network, which will result in an homogeneous film (i.e., uniform appearance and composition).

The term poly(acrylic) acid or acrylic acid polymer is used to encompass a variety of polymers having high percentages of polymerizable monomers therein with pendant carboxylic acid groups or anhydrides of polycarboxylic acid. These are described in more detail in U.S. Pat. Nos. 2,798,053; 3,915,921; 4,267,103; 5,288,814; and 5,349,030. The term polyacrylic acid is used to include various homopolymers, copolymers, and interpolymers, wherein at least 50 or 75 mole percent of the repeating units have pendant carboxylic acid groups or anhydrides of dicarboxylic acid groups. While acrylic acid is the most common primary monomer used to form polyacrylic acid the term is not limited thereto but includes generally all α-β unsaturated monomers with carboxylic pendant groups or anhydrides of dicarboxylic acids as described in U.S. Pat. No. 5,349,030.

The carboxyl containing polymers are prepared from monomers containing at least one activated >C=C< group and carboxyl group. Such polymers are homopolymers of an unsaturated, polymerizable carboxylic monomers such as acrylic acid, methacrylic acid, maleic acid, itaconic acid, maleic anhydride, and the like, and copolymers of polymerizable carboxylic monomers with acrylate esters, acrylamides, olefins, vinyl esters, vinyl ethers, or styrenics. The carboxyl containing polymers have molecular weights greater than about 500 to as high as several million, usually greater than about 10,000 to 900,000 or more.

Copolymers, for example, include copolymers of acrylic acid with small amounts of polyalkenyl polyether cross-linkers that are gel-like polymers, which, especially in the form of their salts, absorb large quantities of water or solvents with subsequent substantial increase in volume. Other useful carboxyl containing polymers are described in U.S. Pat. No. 3,940, 351, directed to polymers of unsaturated carboxylic acid and at least one alkyl acrylic or methacrylic ester where the alkyl group contains 10 to 30 carbon atoms, and U.S. Pat. Nos. 5,034,486; 5, 034,487; and 5,034,488; which are directed to maleic anhydride copolymers with vinyl ethers. Other types of such copolymers are described in U.S. Pat. No. 4,062,817 wherein the polymers described in U. S. Pat. No. 3,940,351 contain additionally another alkyl acrylic or methacrylic ester and the alkyl groups contain 1 to 8 carbon atoms. Carboxylic polymers and copolymers such as those of acrylic acid and methacrylic acid also may be cross-linked with polyfunctional materials as divinyl benzene, unsaturated diesters and the like, as is disclosed in U.S. Pat. Nos. 2, 340,110; 2, 340, 111; and 2,533,635.

The carboxylic monomers are the olefinically-unsaturated carboxylic acids containing at least one activated carbon-to-carbon olefinic double bond, and at least one carboxyl group; that is, an acid or function readily converted to an acid containing an olefinic double bond which readily functions in polymerization because of its presence in the monomer molecule, either in the alpha-beta position with respect to a carboxyl group,--C=C--COOH; or as part of a terminal methylene grouping, CH₂=C<. Olefinically-unsaturated acids of this class include such materials as the acrylic acids typified by the acrylic acid itself, alpha-cyano acrylic acid, beta methylacrylic acid (crotonic acid), alpha-phenyl acrylic acid, beta-acryloxy propionic acid, cinnamic acid, p-chloro cinnamic acid, 1-carboxy-4-phenyl butadiene-1,3, itaconic acid, citraconic acid, mesaconic acid, glutaconic acid, aconitic acid, maleic acid, fumaric acid, and tricarboxy ethylene. As used herein, the term "carboxylic acid" includes the polycarboxylic acids and those acid anhydrides, such as maleic anhydride, wherein the anhydride group is formed by the elimination of one molecule of water from two carboxyl groups located on the same carboxylic acid molecule. Maleic anhydride and other acid anhydrides useful herein have the general structure 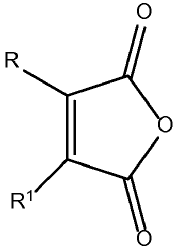 wherein R and R' are selected from the group consisting of hydrogen, halogen and cyanogen (--C≡N) groups and alkyl, aryl, alkaryl, aralkyl, and cycloalkyl groups such as methyl, ethyl, propyl, octyl, decyl, phenyl, tolyl, xylyl, benzyl, cyclohexyl, and the like.

The preferred carboxylic monomers are the monoolefinic acrylic acids having the general structure: 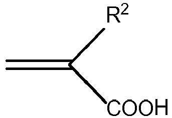 wherein R² is a substituent selected from the class consisting of hydrogen, halogen, and the cyanogen (--C≡N) groups, monovalent alkyl radicals, monovalent aryl radicals, monovalent aralkyl radicals, monovalent alkaryl radicals and monovalent cycloaliphatic radicals. Of this class, acrylic and methacrylic acid are most preferred. Other useful carboxylic monomers are maleic acid and its anhydride.

The polymers include both homopolymers of carboxylic acids or anhydrides thereof, or the defined carboxylic acids copolymerized with one or more other vinylidene monomers containing at least one terminal >C=CH₂ group. The other vinylidene monomers are present in an amount of less than 30 weight percent based upon the weight of the carboxylic acid or anhydride plus the vinylidene monomer(s). Such monomers include, for example, acrylate ester monomers including those acrylic acid ester monomers such as derivatives of an acrylic acid represented by the formula 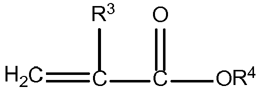 wherein R³ is an alkyl group having from 1 to 30 carbon atoms, preferably 1 to 20 carbon atoms and R⁴ is hydrogen, methyl or ethyl, present in the copolymer in amount, for example, from about 1 to 40 weight percent or more. Representative acrylates include methyl acrylate, ethyl acrylate, propyl acrylate, isopropyl acrylate, butyl acrylate, isobutyl acrylate, methyl methacrylate, methyl ethacrylate, ethyl methacrylate, octyl acrylate, heptyl acrylate, octyl methacrylate, isopropyl methacrylate, 2-ethylhexyl methacrylate, nonyl acrylate, hexyl acrylate, n-hexyl methacrylate, and the like. Higher alkyl acrylic esters are decyl acrylate, isodecyl methacrylate, lauryl acrylate, stearyl acrylate, behenyl acrylate and melissyl acrylate. Mixtures of two or three or more long chain acrylic esters may be successfully polymerized with one of the carboxylic monomers. Other comonomers include olefins, including alpha olefins, vinyl ethers, vinyl esters, and mixtures thereof.

The polymers also may be cross-linked with any polyene, e.g. decadiene or trivinyl cyclohexane; acrylamides, such as methylene bis acrylamide; polyfunctional acrylates, such as trimethylol propane triacrylate; or polyfunctional vinylidene monomer containing at least 2 terminal CH₂ =C< groups, including for example, butadiene, isoprene, divinyl benzene, divinyl naphthlene, allyl acrylates and the like. Particularly useful cross-linking monomers for use in preparing the copolymers are polyalkenyl polyethers having more than one alkenyl ether grouping per molecule. The most useful possess alkenyl groups in which an olefinic double bond is present attached to a terminal methylene grouping, CH₂ =C<. They are made by the etherification of a polyhydric alcohol containing at least 2 carbon atoms and at least 2 hydroxyl groups. Compounds of this class may be produced by reacting an alkenyl halide, such as allyl chloride or allyl bromide, with a strongly alkaline aqueous solution of one or more polyhydric alcohols. The product may be a complex mixture of polyethers with varying numbers of ether groups. Analysis reveals the average number of ether groupings on each molecule. Efficiency of the polyether cross-linking agent increases with the number of potentially polymerizable groups on the molecule. It is preferred to utilize polyethers containing an average of two or more alkenyl ether groupings per molecule. Other cross-linking monomers include for example, diallyl esters, dimethallyl ethers, allyl or methallyl acrylates and acrylamides, tetraallyl tin, tetravinyl silane, polyalkenyl methanes, diacrylates, and dimethacrylates, divinyl compounds such as divinyl benzene, divinyl glycol, polyallyl phosphate, diallyloxy compounds and phosphite esters and the like. Typical agents are allyl pentaerythritol, allyl sucrose, trimethylolpropane triacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol dimethacrylate, and the like. Allyl pentaerythritol, trimethylolpropane diallylether and allyl sucrose provide excellent polymers. When the cross-linking agent is present, the polymeric mixtures usually contain up to about 5% or less by weight of cross-linking monomer based on the total of carboxylic acid monomer, plus other monomers, if present, and more preferably about 0.01 to 3.0 weight percent.

Other vinylidene monomers may also be used, including the acrylic nitriles. The useful α,β-olefinically unsaturated nitriles are preferably the monoolefinically unsaturated nitriles having from 3 to 10 carbon atoms such as acrylonitrile, methacrylonitrile, and the like. Most preferred are acrylonitrile and methacrylonitrile. The amounts used are, for example, for some polymers are from about 1 to 30 weight percent of the total monomers copolymerized. Acrylic amides containing from 3 to 35 carbon atoms including monoolefinically unsaturated amides also may be used. Representative amides include acrylamide, methacrylamide, N-t-butyl acrylamide, N-cyclohexyl acrylamide, higher alkyl amides, where the alkyl group on the nitrogen contains from 8 to 32 carbon atoms, acrylic amides including N-alkylol amides of alpha, beta-olefinically unsaturated carboxylic acids including those having from 4 to 10 carbon atoms such as N-methylol acrylamide, N-propanol acrylamide, N-methylol methacrylamide, N-methylol maleimide, N-methylol maleamic acid esters, N-methylol-p-vinyl benzamide, and the like. Still further useful materials are alpha-olefins containing from 2 to 18 carbon atoms, more preferably from 2 to 8 carbon atoms; dienes containing from 4 to 10 carbon atoms; vinyl esters and allyl esters such as vinyl acetate; vinyl aromatics such as styrene, methyl styrene and chlorostyrene; vinyl and allyl ethers and ketones such as vinyl methyl ether and methyl vinyl ketone; chloroacrylates; cyanoalkyl acrylates such as α-cyanomethyl acrylate, and the α-, β-, and γ- cyanopropyl acrylates; alkoxyacrylates such as methoxy ethyl acrylate; haloacrylates as chloroethyl acrylate; vinyl halides and vinyl chloride, vinylidene chloride and the like; divinyls, diacrylates and other polyfunctional monomers such as divinyl ether, diethylene glycol diacrylate, ethylene glycol dimethacrylate, ethylene-bisacrylamide, allylpentaerythritol, and the like; and bis (β-haloalkyl) alkenyl phosphonates such as bis(β-chloroethyl) vinyl phosphonate and the like as are known to those skilled in the art. Copolymers wherein the carboxy containing monomer is a minor constituent, and the other vinylidene monomers present as major components are readily prepared in accordance with the process of this invention.

The steric stabilizer functions to provide a steric barrier which repulses approaching particles. A requirement for the steric stabilizer is that a segment of the dispersant (i.e., a hydrophobe) be very soluble in the solvent (the continuous phase in a nonaqueous dispersion polymerization process) and that another segment (i.e., a hydrophile) be at least strongly adhered to the growing polymer particle. Thus, the steric stabilizers of the present invention have a hydrophilic group and a hydrophobic group. The steric stabilizers are block copolymers comprising a soluble block and an anchor block having a molecular weight (i.e., chain length) usually well above 1000, but a hydrophobe length of more than 50 Angstroms, as calculated by the Law of Cosines. These dimensions are determined on the extended configuration using literature values for bond lengths and angles. Thus the steric stabilizers of the present invention are distinguishable from the prior art steric surfactants which may be block copolymers, but have hydrophobe lengths of less than 50 Angstroms. The steric stabilizer of the present invention has either a linear block or a comb configuration, and has a hydrophobe of sufficient length to provide a sufficient steric barrier.

When the steric stabilizer is a linear block copolymeric steric stabilizer, it is defined by the following formula: 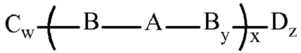 where A is a hydrophilic moiety, having a solubility in water at 25 °C of 1% or greater, a molecular weight of from about 200 to about 50,000, and selected to be covalently bonded to the B blocks;
B is a hydrophobic moiety, having a molecular weight of from about 300 to about 60,000, a solubility of less than 1% in water at 25 °C, capable of being covalently bonded to the A blocks;
and D are terminating groups which can be A or B; can be the same or different groups, and will depend upon the manufacturing process since they are present to control the polymer length, to add other functionality, or as a result of the manufacturing process;
w is 0 or 1;
x is an integer of 1 or more,
y is 0 or 1, and
z is 0 or 1.

Examples of hydrophilic groups are polyethylene oxide, poly(1,3-dioxolane), copolymers of polyethylene oxide or poly(1,3-dioxolane), poly(2-methyl-2-oxazoline polyglycidyl trimethyl ammonium chloride, polymethylene oxide, and the like, with polyethylene oxide being preferred. Examples of hydrophobic groups are polyesters, such as those derived from 2-hydroxybutyric acid, 3-hydroxybutyric acid, 4-hydroxybutyric acid, 2-hydroxycaproic acid, 10-hydroxydecanoic acid, 12-hydroxydodecanoic acid, 16-hydroxyhexadecanoic acid, 2-hydroxyisobutyric acid, 2-(4-hydroxyphenoxy) propionic acid, 4-hydroxyphenylpyruvic acid, 12-hydroxystearic acid, 2-hydroxyvaleric acid, polylactones, such as caprolactone, butyrolactone, polylactams, such as those derived from caprolactam, polyurethanes, polyisobutylene, where the hydrophobe should provide a steric barrier of greater than 50 Angstroms, preferably greater than 75 Angstroms, with greater than 100 Angstroms being also preferred, and the like, with polyhydroxy fatty acids, such as poly(12-hydroxystearic acid) being preferred. The steric barrier is the length of the hydrophobe in its fully-extended condition. Such steric stabilizers are commercially available under the brand name Hypermer® from Croda.

Steric stabilizer molecules comprise both hydrophilic and hydrophobic units. Hydrophobic polymer units or hydrophobic blocks may be prepared by a number of well-known methods. These methods include condensation reactions of hydroxy acids, condensation of polyols (preferably diols) with polycarboxylic acids (preferably diacids). Other useful methods include polymerization of lactones and lactams, and reactions of polyols with polyisocyanates. Hydrophobic blocks or polymer units can be reacted with hydrophilic units by such reactions as are known to those skilled in the art. These reactions include condensation reactions and coupling reactions, for example. Subsequent to the steric stabilizer preparation, the stabilizers may be further reacted with modifying agents to enhance their utility. U.S. Pat. No. 4,203,877 to Alan S. Baker teaches making such steric stabilizers.

When the steric stabilizer is a random copolymeric comb steric stabilizer, it is defined by the following formula:

R⁵-(Z)m-(Q)n-R⁶,

where R⁵ and R⁶ are terminating groups and may be the same or different and will be different from Z and Q,
Z is a hydrophobic moiety having a solubility of less than 1% in water at 25 °C,
Q is a hydrophilic moiety, having a solubility of more than 1% in water at 25 °C,
m and n are integers of 1 or more, and are selected such that the molecular weight of the polymer is from about 100 to about 250,000.

Examples of the hydrophobic monomer unit or moiety are dimethyl siloxane, diphenyl siloxane, methylphenyl siloxane, alkyl acrylate, alkyl methacrylate, and the like, with dimethyl siloxane being preferred.

Examples of the hydrophilic monomer unit or moiety are methyl-3-polyethoxypropyl siloxane-Ω-phosphate or sulfate, and the alkali metal or ammonium salts derived therefrom; units derived from polyethoxy (meth) acrylate containing from 1 to 40 moles of ethylene oxide; acrylic acid; acrylamide; methacrylic acid, maleic anhydride; dimethyl amino ethyl (meth)acrylate; or its salts with methyl chloride or dimethyl sulfate; dimethyl amino propyl(meth)acrylamide and its salts with methyl chloride or dimethyl sulfate, and the like, with methyl-3-polyethoxypropyl siloxane-Ω-phosphate being preferred.

Examples of terminating agents are monohalo silanes, mercaptans, haloalkanes, alkyl aromatics, alcohols, and the like, which will produce terminating groups such as trialkyl silyl, alkyl, aryl alkyl, alcoholate, and the like, with the preferred terminating groups being trimethyl silyl.

Specific types of cross-linked polyacrylic acids include Carbopol® 981NF; Carbopol® 980NF; Pemulen TR2; and carbomer interpolymer ETD-2020-NF; copolymers of acrylic acid and alkyl acrylates; copolymers of acrylic acid and alkyl vinyl ethers; and copolymers of ethylene and maleic anhydride. An approved polyacrylic acid for pharmaceutical applications are carbomer homopolymers, carbomer copolymers, carbomerl interpolymers or polycarbophil, as described in the carbomer and polycarbophil compendia monographs in the U.S.

The TPU compositions described herein are made using a) a polyisocyanate component. The polyisocyanate and/or polyisocyanate component includes one or more polyisocyanates. In some embodiments, the polyisocyanate component includes one or more diisocyanates.

In some embodiments, the polyisocyanate and/or polyisocyanate component includes an α, ω-alkylene diisocyanate having from 5 to 20 carbon atoms.

Suitable polyisocyanates include aromatic diisocyanates, aliphatic diisocyanates, or combinations thereof. In some embodiments, the polyisocyanate component includes one or more aromatic diisocyanates. In some embodiments, the polyisocyanate component is essentially free of, or even completely free of, aliphatic diisocyanates. In other embodiments, the polyisocyanate component includes one or more aliphatic diisocyanates. In some embodiments, the polyisocyanate component is essentially free of, or even completely free of, aromatic diisocyanates.

Examples of useful polyisocyanates include aromatic diisocyanates such as 4,4'-methylenebis(phenyl isocyanate) (MDI), m-xylene diisocyanate (XDI), phenylene-1,4-diisocyanate, naphthalene-1,5-diisocyanate, and toluene diisocyanate (TDI); as well as aliphatic diisocyanates such as isophorone diisocyanate (IPDI), 1,4-cyclohexyl diisocyanate (CHDI), decane-1,10-diisocyanate, lysine diisocyanate (LDI), 1,4-butane diisocyanate (BDI), isophorone diisocyanate (PDI), 3,3'-dimethyl-4,4'-biphenylene diisocyanate (TODI), 1,5-naphthalene diisocyanate (NDI), and dicyclohexylmethane-4,4'-diisocyanate (H12MDI). Mixtures of two or more polyisocyanates may be used. In some embodiments, the polyisocyanate is MDI and/or H12MDI. In some embodiments, the polyisocyanate includes MDI. In some embodiments, the polyisocyanate includes H12MDI.

In some embodiments, the thermoplastic polyurethane is prepared with a polyisocyanate component that includes H12MDI. In some embodiments, the thermoplastic polyurethane is prepared with a polyisocyanate component that consists essentially of H12MDI. In some embodiments, the thermoplastic polyurethane is prepared with a polyisocyanate component that consists of H12MDI.

In some embodiments, the thermoplastic polyurethane is prepared with a polyisocyanate component that includes (or consists essentially of, or even consists of) H12MDI and at least one of MDI, HDI, TDI, IPDI, LDI, BDI, PDI, CHDI, TODI, and NDI.

In some embodiments, the polyisocyanate used to prepare the TPU and/or TPU compositions described herein is at least 50%, on a weight basis, a cycloaliphatic diisocyanate. In some embodiments, the polyisocyanate includes an α, ω-alkylene diisocyanate having from 5 to 20 carbon atoms.

In some embodiments, the polyisocyanate used to prepare the TPU and/or TPU compositions described herein includes hexamethylene-1,6-diisocyanate, 1,12-dodecane diisocyanate, 2,2,4-trimethyl-hexamethylene diisocyanate, 2,4,4-trimethyl-hexamethylene diisocyanate, 2-methyl-1,5-pentamethylene diisocyanate, or combinations thereof.

### The Polyol Component

The TPU compositions described herein are made using b) a polyol component. Polyols include polyether polyols, polyester polyols, polycarbonate polyols, polysiloxane polyols, and combinations thereof.

Suitable polyols, which may also be described as hydroxyl terminated intermediates, when present, may include one or more hydroxyl terminated polyesters, one or more hydroxyl terminated polyethers, one or more hydroxyl terminated polycarbonates, one or more hydroxyl terminated polysiloxanes, polyether/polyester blocks, or mixtures thereof.

Suitable hydroxyl terminated polyester intermediates include linear polyesters having a number average molecular weight (Mn) of from about 500 to about 10,000, from about 700 to about 5,000, or from about 700 to about 4,000, and generally have an acid number less than 1.3 or less than 0.5. The molecular weight is determined by assay of the terminal functional groups and is related to the number average molecular weight. The polyester intermediates may be produced by (1) an esterification reaction of one or more glycols with one or more dicarboxylic acids or anhydrides or (2) by transesterification reaction, i.e., the reaction of one or more glycols with esters of dicarboxylic acids. Mole ratios generally in excess of more than one mole of glycol to acid are preferred so as to obtain linear chains having a preponderance of terminal hydroxyl groups. The dicarboxylic acids of the desired polyester can be aliphatic, cycloaliphatic, aromatic, or combinations thereof. Suitable dicarboxylic acids which may be used alone or in mixtures generally have a total of from 4 to 15 carbon atoms and include: succinic, glutaric, adipic, pimelic, suberic, azelaic, sebacic, dodecanedioic, isophthalic, terephthalic, cyclohexane dicarboxylic, and the like. Anhydrides of the above dicarboxylic acids such as phthalic anhydride, tetrahydrophthalic anhydride, or the like, can also be used. Adipic acid is a preferred acid. The glycols which are reacted to form a desirable polyester intermediate can be aliphatic, aromatic, or combinations thereof, including any of the glycols described above in the chain extender section, and have a total of from 2 to 20 or from 2 to 12 carbon atoms. Suitable examples include ethylene glycol, 1,2-propanediol, 1,3-propanediol, 1,3-butanediol, 1,4-butanediol, 1,5-pentanediol, 1,6-hexanediol, 2,2-dimethyl-1,3-propanediol, 1,4-cyclohexanedimethanol, decamethylene glycol, dodecamethylene glycol, and mixtures thereof.

The polyol component may also include one or more polycaprolactone polyester polyols. The polycaprolactone polyester polyols useful in the technology described herein include polyester diols derived from caprolactone monomers. The polycaprolactone polyester polyols are terminated by primary hydroxyl groups. Suitable polycaprolactone polyester polyols may be made from ε-caprolactone and a bifunctional initiator such as diethylene glycol, 1,4-butanediol, or any of the other glycols and/or diols listed herein. In some embodiments, the polycaprolactone polyester polyols are linear polyester diols derived from caprolactone monomers.

Useful examples include CAPA™ 2202A, a 2,000 number average molecular weight (Mn) linear polyester diol, and CAPA™ 2302A, a 3,000 Mn linear polyester diol, both of which are commercially available from Perstorp Polyols Inc. These materials may also be described as polymers of 2-oxepanone and 1,4-butanediol.

The polycaprolactone polyester polyols may be prepared from 2-oxepanone and a diol, where the diol may be 1,4-butanediol, diethylene glycol, monoethylene glycol, 1,6-hexanediol, 2,2-dimethyl-1,3-propanediol, or any combination thereof. In some embodiments, the diol used to prepare the polycaprolactone polyester polyol is linear. In some embodiments, the polycaprolactone polyester polyol is prepared from 1,4-butanediol. In some embodiments, the polycaprolactone polyester polyol has a number average molecular weight from 500 to 10,000, or from 500 to 5,000, or from 1,000 or even 2,000 to 4,000 or even 3,000.

Suitable hydroxyl terminated polyether intermediates include polyether polyols derived from a diol or polyol having a total of from 2 to 15 carbon atoms, in some embodiments an alkyl diol or glycol which is reacted with an ether comprising an alkylene oxide having from 2 to 6 carbon atoms, typically ethylene oxide or propylene oxide or mixtures thereof. For example, hydroxyl functional polyether can be produced by first reacting propylene glycol with propylene oxide followed by subsequent reaction with ethylene oxide. Primary hydroxyl groups resulting from ethylene oxide are more reactive than secondary hydroxyl groups and thus are preferred. Useful commercial polyether polyols include poly(ethylene glycol) comprising ethylene oxide reacted with ethylene glycol, poly(propylene glycol) comprising propylene oxide reacted with propylene glycol, poly(tetramethylene ether glycol) comprising water reacted with tetrahydrofuran which can also be described as polymerized tetrahydrofuran, and which is commonly referred to as PTMEG. In some embodiments, the polyether intermediate includes PTMEG. Suitable polyether polyols also include polyamide adducts of an alkylene oxide and can include, for example, ethylenediamine adduct comprising the reaction product of ethylenediamine and propylene oxide, diethylenetriamine adduct comprising the reaction product of diethylenetriamine with propylene oxide, and similar polyamide type polyether polyols. Copolyethers can also be utilized in the described compositions. Typical copolyethers include the reaction product of THF and ethylene oxide or THF and propylene oxide. These are available from BASF as PolyTHF® B, a block copolymer, and PolyTHF® R, a random copolymer. The various polyether intermediates generally have a number average molecular weight (Mn) as determined by assay of the terminal functional groups which is an average molecular weight greater than about 300, or greater than about 1450, such as from about 700 to about 10,000, from about 1,450 to about 5,000, or from about 1,450 to about 2,500. In some embodiments, the polyether intermediate includes a blend of two or more different molecular weight polyethers, such as a blend of 300 Mn and 8,000 Mn PEG or a blend of 300 Mn, 1450 Mn and 8,000 Mn PEG.

Suitable hydroxyl terminated polycarbonates include those prepared by reacting a glycol with a carbonate. U.S. Patent No. 4,131,731 discloses hydroxyl terminated polycarbonates and their preparation. Such polycarbonates are linear and have terminal hydroxyl groups with essential exclusion of other terminal groups. The essential reactants are glycols and carbonates. Suitable glycols are selected from cycloaliphatic and aliphatic diols containing 4 to 40, and or even 4 to 12 carbon atoms, and from polyoxyalkylene glycols containing 2 to 20 alkoxy groups per molecule with each alkoxy group containing 2 to 4 carbon atoms. Suitable diols include aliphatic diols containing 4 to 12 carbon atoms such as 1,4-butanediol, 1,5-pentanediol, neopentyl glycol, 1,6-hexanediol, 2,2,4-trimethyl-1,6-hexanediol, 1,10-decanediol, hydrogenated dilinoleylglycol, hydrogenated dioleylglycol, 3-methyl-1,5-pentanediol; and cycloaliphatic diols such as 1,3-cyclohexanediol, 1,4-dimethylolcyclohexane, 1,4-cyclohexanediol-, 1,3-dimethylolcyclohexane-, 1,4-endomethylene-2-hydroxy-5-hydroxymethyl cyclohexane, and polyalkylene glycols. The diols used in the reaction may be a single diol or a mixture of diols depending on the properties desired in the finished product. Polycarbonate intermediates which are hydroxyl terminated are generally those known to the art and in the literature. Suitable carbonates are selected from alkylene carbonates composed of a 5 to 7 member ring. Suitable carbonates for use herein include ethylene carbonate, trimethylene carbonate, tetramethylene carbonate, 1,2-propylene carbonate, 1,2-butylene carbonate, 2,3-butylene carbonate, 1,2-ethylene carbonate, 1,3-pentylene carbonate, 1,4-pentylene carbonate, 2,3-pentylene carbonate, and 2,4-pentylene carbonate. Also, suitable herein are dialkylcarbonates, cycloaliphatic carbonates, and diarylcarbonates. The dialkylcarbonates can contain 2 to 5 carbon atoms in each alkyl group and specific examples thereof are diethylcarbonate and dipropylcarbonate. Cycloaliphatic carbonates, especially dicycloaliphatic carbonates, can contain 4 to 7 carbon atoms in each cyclic structure, and there can be one or two of such structures. When one group is cycloaliphatic, the other can be either alkyl or aryl. On the other hand, if one group is aryl, the other can be alkyl or cycloaliphatic. Examples of suitable diarylcarbonates, which can contain 6 to 20 carbon atoms in each aryl group, are diphenylcarbonate, ditolylcarbonate, and dinaphthylcarbonate.

Suitable polysiloxane polyols include α-ω-hydroxyl or amine or carboxylic acid or thiol or epoxy terminated polysiloxanes. Examples include poly(dimethysiloxane) terminated with a hydroxyl or amine or carboxylic acid or thiol or epoxy group. In some embodiments, the polysiloxane polyols are hydroxyl terminated polysiloxanes. In some embodiments, the polysiloxane polyols have a number-average molecular weight in the range from 300 to 5,000, or from 400 to 3,000.

Polysiloxane polyols may be obtained by the dehydrogenation reaction between a polysiloxane hydride and an aliphatic polyhydric alcohol or polyoxyalkylene alcohol to introduce the alcoholic hydroxy groups onto the polysiloxane backbone.

In some embodiments, the polysiloxanes may be represented by one or more compounds having the following formula: 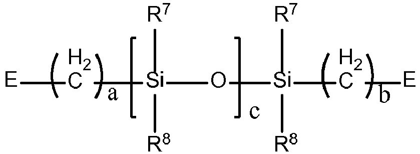 in which: each R⁷ and R⁸ are independently a 1 to 4 carbon atom alkyl group, a benzyl, or a phenyl group; each E is OH or NHR³ where R³ is hydrogen, a 1 to 6 carbon atoms alkyl group, or a 5 to 8 carbon atoms cyclo-alkyl group; a and b are each independently an integer from 2 to 8; c is an integer from 3 to 50. In amino-containing polysiloxanes, at least one of the E groups is NHR³. In the hydroxyl-containing polysiloxanes, at least one of the E groups is OH. In some embodiments, both R¹ and R² are methyl groups.

Suitable examples include α,ω-hydroxypropyl terminated poly(dimethysiloxane) and α,ω-amino propyl terminated poly(dimethysiloxane), both of which are commercially available materials. Further examples include copolymers of the poly(dimethysiloxane) materials with a poly(alkylene oxide).

The polyol component, when present, may include poly(ethylene glycol), poly(tetramethylene ether glycol), poly(trimethylene oxide), ethylene oxide capped poly(propylene glycol), poly(butylene adipate), poly(ethylene adipate), poly(hexamethylene adipate), poly(tetramethylene-co-hexamethylene adipate), poly(3-methyl-1,5-pentamethylene adipate), polycaprolactone diol, poly(hexamethylene carbonate) glycol, poly(pentamethylene carbonate) glycol, poly(trimethylene carbonate) glycol, dimer fatty acid based polyester polyols, vegetable oil based polyols, or any combination thereof.

Examples of dimer fatty acids that may be used to prepare suitable polyester polyols include Priplast™ polyester glycols/polyols commercially available from Croda and Radia® polyester glycols commercially available from Oleon.

In some embodiments, the polyol component includes a polyether polyol, a polycarbonate polyol, a polycaprolactone polyol, or any combination thereof.

In some embodiments, the polyol component includes a polyether polyol. In some embodiments, the polyol component is essentially free of or even completely free of polyester polyols. In some embodiments, the polyol component used to prepare the TPU is substantially free of, or even completely free of polysiloxanes.

In some embodiments, the polyol component includes ethylene oxide, propylene oxide, butylene oxide, styrene oxide, poly(tetramethylene ether glycol), poly(propylene glycol), poly(ethylene glycol), copolymers of poly(ethylene glycol) and poly(propylene glycol), epichlorohydrin, and the like, or combinations thereof. In some embodiments the polyol component includes poly(ethylene glycol).

The polyol component, in some embodiments, may include a multi-block polyol. The multi-block polyol can include combinations of polyether with polyester, for example, polyethylene oxide polyether (PEO)-polycaprolactone (PCL)) or (PCL-PEO-PCL) which give good control over hydrophilicity, degradation and mechanical properties. The use of multiblock polyether products PEO-PPO (polypropylene oxide -PEO better known as Pluronics® (a registered trademark of BASF Corporation) and block polyester such as PCL-PEO-PPO-PEO-PCL may also be used. It is also contemplated that alternative ester and ether blocks may be used, for example, multiblock polyethers in combination with a block polyester.

### The Chain Extender Component

The TPU compositions described herein are made using c) a chain extender component. Chain extenders include diols, diamines, and combination thereof.

Suitable chain extenders include relatively small polyhydroxy compounds, for example lower aliphatic or short chain glycols having from 2 to 20, or 2 to 12, or 2 to 10 carbon atoms. Suitable examples include ethylene glycol, diethylene glycol, propylene glycol, dipropylene glycol, 1,4-butanediol (BDO), 1,6-hexanediol (HDO), 1,3-butanediol, 1,5-pentanediol, neopentylglycol, 1,4-cyclohexanedimethanol (CHDM), 2,2-bis[4-(2-hydroxyethoxy) phenyl]propane (HEPP), hexamethylenediol, heptanediol, nonanediol, dodecanediol, 3-methyl-1,5-pentanediol, ethylenediamine, butanediamine, hexamethylenediamine, and hydroxyethyl resorcinol (HER), and the like, as well as mixtures thereof. In some embodiments the chain extender includes BDO, HDO, 3-methyl-1,5-pentanediol, or a combination thereof. In some embodiments, the chain extender includes BDO. Other glycols, such as aromatic glycols could be used, but in some embodiments the TPUs described herein are essentially free of or even completely free of such materials.

In some embodiments, the chain extender used to prepare the TPU is substantially free of, or even completely free of, 1,6-hexanediol. In some embodiments, the chain extender used to prepare the TPU includes a cyclic chain extender. Suitable examples include CHDM, HEPP, HER, and combinations thereof. In some embodiments, the chain extender used to prepare the TPU includes an aromatic cyclic chain extender, for example HEPP, HER, or a combination thereof. In some embodiments, the chain extender used to prepare the TPU includes an aliphatic cyclic chain extender, for example CHDM. In some embodiments, the chain extender used to prepare the TPU is substantially free of, or even completely free of aromatic chain extenders, for example aromatic cyclic chain extenders. In some embodiments, the chain extender used to prepare the TPU is substantially free of, or even completely free of polysiloxanes.

In some embodiments, the chain extender component includes 1,4-butanediol, 2-ethyl-1,3-hexanediol, 2,2,4-trimethyl pentane-1,3-diol, 1,6-hexanediol, 1,4-cyclohexane dimethylol, 1,3-propanediol, 3-methyl-1,5-pentanediol or combinations thereof. In some embodiments, the chain extender component includes 1,4-butanediol, 3-methyl-1,5-pentanediol or combinations thereof. In some embodiments, the chain extender component includes 1,4-butanediol.

### Additional TPU Components

In some embodiments, the TPU described herein will further include an optional chain terminating agent. Chain terminating agents are well known and may be a monohydroxyl or mono primary amine or any other mono function compound that reacts with a di-isocyanate to terminate the step growth polymerization at the end of the polymerchain. These may be the same or different on either end of the polymer. The chain terminating agent may have a number average molecular weight ranging from 100 to 8000, linked to the polymer via a urethane or urea bond.

Examples of chain terminating agents include mono amine- or mono alcohol-terminated polyalkylene oxides, silicones, alkyl, alkylesters, polyalkylene esters and mixtures thereof. In some embodiments, a chain terminating group that may be used in the polyurethane copolymers according to the present invention include monofunctional polyethylene oxides, monofunctional polytetramethylene oxides, monofunctional polypropylene oxides, monofunctional siloxanes, and mixtures and/or copolymers thereof. Dodecylamines, alkoxylated alcohols such as cetereth-20, steareth 20 and the like. In one embodiment, the amount of chain terminating agent is from 0 wt%-2 weight% based on the total weight of the dry polyurethane copolymer.

The composition described herein is generally formed by dispersing a crosslinked poly(acrylic) acid polymer into water or a miscible water/organic solvent mixture. The amount of the crosslinked poly(acrylic) acid polymer is, in one embodiment, from about 0.1 parts to about 6.5 parts by weight and in another embodiment, from about 1 part to about 3 parts by weight for every 100 parts by weight of water or miscible water/organic solvent mixture. The hydrophilic TPU polymer is dissolved, in one embodiment, in an organic solvent or miscible organic solvent/water mixture in an amount of from about 1 to about 40 parts by weight and desirably from about 3 to about 15 parts by weight for every 100 parts by weight of solvent or miscible organic solvent/water mixture. In another embodiment, the hydrophilic TPU is dissolved in a solvent:water solution in a weight ratio of 90:10 to 10:90. Suitable solvents include ethanol, tetrahydrofuran (THF), dimethylforamide (DMF), isopropyl alcohol (IPA), acetone, and combinations of these solvents with water in a weight ratio of 90:10 to 10:90.

It has been found that the degree of neutralization of the poly(acrylic) acid polymer has a direct impact on preparation of the blended poly(acrylic) acid polymer and TPU as well as the final film properties. Accordingly, in one embodiment, prior to blending of the two polymers, the poly(acrylic) acid polymer is partially neutralized from an initial pH of from about 2.0 to about 4.0 or from about 2.5 to about 3.5 or about 3.0. In one embodiment the amount of neutralizer used is from 25% to 50% of the theoretical value necessary to achieve a polymer solution of pH 7. In another embodiment, the amount of neutralization is from 10% to 75% of the acid content of the polymer. In a still further embodiment, the pH of the polymer solution is from 4 to 8. Neutralization can be carried out with any convenient neutralizing agent or compound such as ammonium hydroxide, sodium hydroxide, other alkali hydroxides, borates, phosphates, pyrophosphates or polyphosphates; an amino acid, such as arginine; AMP-95 (2-Amino-2-Methyl-1-Propanol) a product of Angus Chemical, cocamine, oleamine, diisopropanolamine, diisopropylamine, dodecylamine, Peg-15 cocoamine, morpholine, tetrakis(hydroxypropyl)ethylenediamine, triamylamine, triethanolamine, triethylamine, or tromethamine (2-Amino 2-Hydroxymethyl-1,3-propanediol). In some embodiments, neutralizing agents include NaOH, tetrakis(hydroxypropyl)ethylenediamine, triethanolamine, and tromethamine.

The poly(acrylic) acid polymer and hydrophilic TPU are then blended together. In one embodiment, the ratio, by weight, of poly(acrylic) acid polymer to hydrophilic TPU is from 1:1 to 1:60, and in another embodiment from 1:1 to 1:20, and in a further embodiment from 1:1 to 1:3. Optionally, additional water or other solvent such as alcohols, polyols, or polyalkoxides can be added. Such additional water or solvent is dependent upon the desired final qualities and physical constraints of individual formulations.

In one embodiment, the composition of the invention can be prepared as a homogenous film. Prior to film casting air bubbles are removed from blends prepared from mixing the partially neutralized poly(acrylic) polymer and TPU by centrifugation. Degassed blends are used to solvent cast films on polyethylene substrates using an automatic film applicator with vacuum plate using a draw bar. Resulting films are dried in air at room temperature.

In some embodiments, the films disclosed herein may be sterilized. Sterilization is the treatment process that rids materials of possible contaminants, including microbial life, bacteria, fungi and viruses. In order to limit transmission of these contaminants, the medical industry requires certain levels of sterilization. Several sterilization methods may be used. In one embodiment, sterilization may be conducted by immersing the product in ethylene oxide gas in a chamber, then aerating it. In another embodiment, the product is put in a sterilization chamber that is vacuumed and filled with hydrogen peroxide vapor and then aerated. Sterilization involving ionizing energy that has low penetration and uses a high dose rate to eliminate contaminants may be used. An accelerator produces a beam of electrons that are focused on the product to be sterilized. Sterilization using an isotope source, usually Cobalt-60, to produce ionizing energy that flows through the product may also be used. This energy causes cellular damage to the organisms, ridding the product of them. Sterilization utilizing hot air, conducting heat through the equipment may be used. Objects are heated to a steady temperature and held for a certain length of time, depending on the material. Dry heat sterilization is very effective, as it can reach all surfaces of an assembled product.

The film as described herein, may, in one embodiment, be partially swollen with respect to the full amount of water the film is capable of absorbing. Such amounts of water absorption are, in one embodiment, from 400% to 3000% by weight of the dry film, or from 600% to 1200% or to 800%.

The film as described herein can be utilized in various forms. In one embodiment, the film is in the form of a patch or a medicated patch, wherein the film is attached to a suitable backing. In one embodiment, the patch may be an adhesive patch. In another embodiment, the patch may be a non-adhesive patch. A facing, such as a release liner, is applied on top of the film. In another embodiment, the film is in the form of a single layer and includes a release layer on either side thereof, or on both sides thereof. The thickness of the singly layer is, in one embodiment, from 0.5 to 100 mil, and in another embodiment from 1 to 50 mil, and in still further embodiment from 1 to 5 mil. The film containing a substance therein, be it a personal care compound, a pharmaceutical compound, an active ingredient, a biological active compound, an absorptive material, etc., can be wrapped in a suitable container such as an impervious plastic wrap, foil pouch, etc. and stored until needed. It can then be applied to a desired substrate, as set forth below.

The applied substance can be any material known, purported, or thought to have a beneficial effect on the chosen substrate, such as the substances listed in the preceding paragraph. While water-soluble active ingredients are most easily incorporated, the use of non-water carriers, emulsifiers, dispersed organic (hydrocarbon) phases, etc., can allow the delivery of nonpolar compounds (e.g., hydrocarbon materials such as aliphatic and aromatic compounds).

One class of substances are the therapeutic aids which include, but are not limited to, moisturizers (or things that help the substrate (skin) retain water); oils (or things that help the skin retain oil); pharmaceutical agents; antimicrobial agents; antibacterial agents; fungicide; anti-inflammatory/analgesic agents (e.g., things that reduce irritation); softening agents; toughening agents; agents that enhance elasticity of the substrate; agents that promote cell growth or cell reproduction; agents that retard cell growth or cell reproduction; stimulants for the cells or nerves, antihistamines; local anesthetics; and the like.

The film may include one or more active ingredients with one or more of the following advantages: sustained delivery, consistency in dosage, enhanced delivery, dosage control, efficiency, and bioavailability for: wound healing, burn healing, scar reducing, etc.; skin or keratin color changes (lightening, darkening, coloring), applying decorative images, highlighting; enhancing penetration of another active ingredient or medicine through the skin or other substrate; altering the fragrance or aroma of the substrate, or enhancing fat, e.g., cellulite reduction; applying a hormone, steroid, or pheromone, etc.

The active ingredient may be of any polarity from low to high including fragrances, coloring, pigments, ointments, etc. Where desired, water solubility may be enhanced by the addition of other carriers, additives, etc. In many embodiments, a mixture of two or more active ingredients which act independently or in conjunction with each other will be used. The active ingredient can be any of the following: a moisturizer, an anti-aging agent (removing aging effect or repairing aging effects); an astringent, an acid (e.g., glycolic, citric, and vitamins); a skin stimulator (e.g., menthol, camphor, and cayenne pepper extract); a firming agent; a slimming agent; a radical scavenger; solubilizers, an antihistamine (e.g., diphenhydramine or chlorpheniramine maleate); methyl salicylate; glycol salicylate; an aroma-therapeutic; a humectant; an emollient; a phytochemical (natural extract such as herbal and botanical e.g., bamboo, tea tree oil, etc.), an antioxidant, a skin whitening agent (e.g., hydroquinone, peroxide, and kojic acid); a self-tanning agent or agent for adding skin colorant (e.g., dihydroxy acetone); a skin protecting agent (e.g., moisturizers, waxes, sunblocks (organic or inorganic)); a spot remover (substrate may be people, clothing, animals, plant, hard surface, or fabric); keratin, retinol; vitamins; vitamin complexes; precursors of active ingredients such a precursors of retinol; salicylic acid and derivatives of salicylic acid; peptide;oligomeric and polymeric peptide; an enzyme; a coenzyme; proteins and their precursors; amino acid (e.g., dimers, cyclic and aliphatic amino acid); glycosamineoglycans; saccharides; derivatives of saccharides; plysaccharides; oligomeric saccharides; cyclic oligomeric saccharides; carbohydrates, fatty acid triglycerides essential fatty acids; lipids; lecithin; phospholipids; conditioning agents; milk derivatives; carotenes; cyclodextrins; tocopherols; phytosterols; cationic agents; oil (natural such a animal and vegetable, synthetic including primrose oil, jojoba oil, mineral oil, castor oil, palm oil, coconut oil, corn oil, silicones, and derivatized forms thereof; gelatins, natural starch, modified starches, cellulosics and chemically modified cellulosics, sodium alginate, acacia, corn starch, cascin, natural gums, and/or modified natural gums; waxes (natural such a plant and synthetic); quaternized compounds; silicone and /or silicone derivatives; protein hydrozylates or derivative proteins; chitin; denatured chitin; chitosan; marine derived compounds or marine origin materials (e.g. anything from the sea including things such as kelp, coral, seawood, marine moisturizing factor, algae, sea plants, phytoplankton, kelp, and their extracts); hydrolyzed animal and/or vegetable protein; astringent (e.g. zinc oxide, tannic acid, alum, aluminum sulfate, vitamin, dl-α-tocopherol); a wetting agent; a water repellant; an antimicrobial; a deodorant; a fungicide; a fruit acid; nut extracts/oils; a fragrance; flower acids; ceramides; a flavonoid; biologically derived materials (biotechnology); sodium hyaluronate; hyaluronic acid; etc.

In one embodiment, the clarity and/or appearance of the films of the invention can be adjusted. The clarity of the films may vary from substantially transparent, with little visual haze, to where insoluble component additives such as beads, air bubbles, pearlizing agents, are clearly visible to visually opaque. The films may incorporate long-term suspension of particles, insoluble liquid droplets, or the stabilization of gas bubbles within the medium. The materials or compounds which may be suspended can be soluble or insoluble. In some embodiments, the film is opacified by deliberately incorporating pearlescent materials therein to achieve an attractive pearl-like appearance, known as pearlescence. Examples of such other insoluble compounds include pigments, minerals such as bismuth, antimicrobials such as silver or zinc particles, dyes, and the like.

Visually distinct, multiple phase compositions where one phase is clear and another phase is opaque are also envisioned. In one embodiment of the invention, a pattern comprising phases that are visually distinct from each other may be formed by mixing clear and opaque components. The visual distinction between each phase can be in color, texture, density, and the type of insoluble component or benefit agent contained therein. The specific pattern can be chosen from a wide variety of patterns.

### Film properties

The choice of the TPU component, and the ratio between the poly(acrylic) acid polymer and the TPU component, as well as the degree of neutralization of the poly(acrylic) acid polymer will each have an impact on the physical properties of the resulting film. These parameters may be used to select the combination of the properties desired in the resulting film. By way of example, the physical properties may include one or more of the following: tensile strength, water absorption, moisture permeability, elongation, stiffness and combinations thereof. A few of the more important physical properties are further commented on below.

Tensile strength can be determined according to ASTM D882-12. In some embodiments the film exhibits a tensile strength in the range of 5 MPa to 50 MPa or 15 MPa to 35 MPa.

Fluid absorption can be determined by weight or by area according to methods described in the art and exemplified in Example 3. The fluid absorption can be expressed as % weight fluid absorbed as compared with the dry film, or as grams of fluid absorbed/gram film, or as grams fluid absorbed/area. In some embodiments, the film exhibits a fluid absorption in the range of 400% to 3000% or from 800% to 1200% or in another embodiment to 800%. This is equivalent to the film exhibiting a fluid absorption in the range of 4 g fluid absorbed/1 g of film to 30 g fluid absorbed/1 g of film or from 8 g fluid absorbed/1 g film to 12 g fluid absorbed/1 g film or to 8 g fluid absorbed/1 g film. This is equivalent to the film exhibiting a fluid absorption from about 0.22 g fluid absorbed/10 cm² to 1.4 g fluid absorbed/10 cm² or from about 0.35 g fluid absorbed/10 cm² to about 0.58 g fluid absorbed/10 cm² or of about 0.35 g fluid absorbed/10 cm².

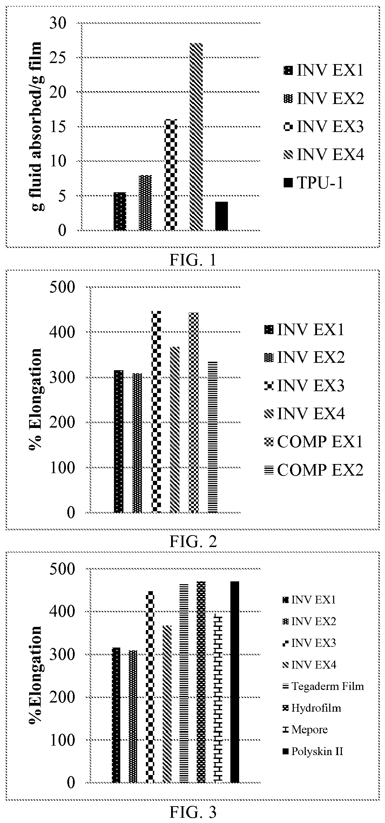

The fluid absorption of the films is illustrated in Fig. 1 in which the grams fluid absorbed per gram of film is shown for Examples 1-4 as compared to a commercially available thermoplastic polyurethane film. As is shown, the films as described herein have increased absorptive capacity as compared to a film formed from a thermoplastic polyurethane only.

Percent elongation can be determined according to ASTM D882-12. In some embodiments, the film exhibits a percent elongation in the range from 100% to 700% or from 300% to 400%. Percent elongation of the films is further illustrated in Fig. 2 in which the percent elongation of the films exemplified in Examples 1-4 as compared with films formed from commercially available thermoplastic polyurethane films. The films of the present invention exhibit percent elongation at least equivalent to or better than the commercially available thermoplastic polyurethane films, as well as commercially available wound dressing films, as shown in Fig. 3.. It should be noted that the commercial wound dressing films contain an adhesive layer in addition to the polyurethane film.

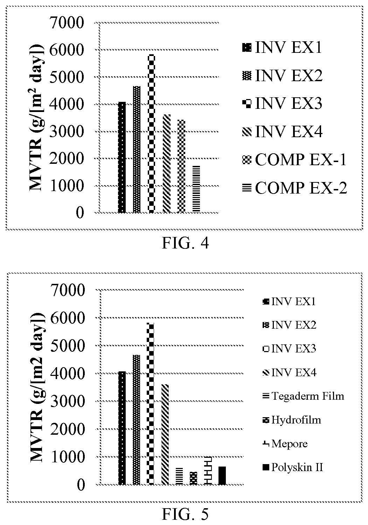

Moisture vapor transmission rate (MVTR) can be determined according to methods described in the art and exemplified in Example 3. In some embodiments, the film exhibits a MVTR of from 1000 to 8000 g/(m²xday) or from 3000 to 5000 g/(m²xday). MVTR of the films described herein is further illustrated in Fig. 4 in which the MVTR of films exemplified in Examples 1-4 is compared to the MVTR of commercially available thermoplastic polyurethane films. Films of the present invention exhibit an increased MVTR over commercially available thermoplastic polyurethane films, as well as compared to commercially available wound dressing films, as illustrated in Fig. 5. It should be noted that the commercial wound dressing films contain an adhesive layer in addition to the polyurethane film.

Stiffness or Young's modulus can be determined according to ASTM D882-12. In some embodiments, the film exhibits a Young's modulus in the range from 3 MPa to 150 MPa or from 5 MPa to 40 MPa or from 10 MPa to 30 MPa.

### Industrial application

The homogenous film described herein may be used in any number of applications and/or articles. Examples include but are not limited to medical applications, for example, where the film described herein may be used in wound management, as well as used in, personal care applications, pharmaceutical applications, health care product applications, or any other number of applications. The term "health care product" as used herein includes, without being limited thereto, pharmaceuticals (controlled release pharmaceuticals), pharmacosmetics and over-the- counter products and appliances (topical and transdermal), such as patches, plasters and the like, externally applied to the body, including the skin, scalp, nails and mucous membranes of humans and animals, for ameliorating a health- related or medical condition, for generally maintaining hygiene or well-being, and the like.

It is also contemplated that the film described herein may be used as an acoustic transmission media, also referred to as an ultrasound couplant, ultrasound gel, or ultrasound transmission media, in which the film is utilized to conduct ultrasound between an electronic device and a target body. The film, in such an application, functions as a hydrogel membrane that is acoustically conductive, provides acceptable low levels of ultrasound impedance and artifact with excellent ultrasound transmission, thus creating membranes that can perform as solid couplants, suitable for medical ultrasound imaging and ultrasound based therapy applications in place of gels and thickened liquids known in the art.

The amount of each chemical component described is presented exclusive of any solvent, which may be customarily present in the commercial material, that is, on an active chemical basis, unless otherwise indicated. However, unless otherwise indicated, each chemical or composition referred to herein should be interpreted as being a commercial grade material which may contain the isomers, by-products, derivatives, and other such materials which are normally understood to be present in the commercial grade.

It is known that some of the materials described above may interact in the final formulation, so that the components of the final formulation may be different from those that are initially added. For instance, metal ions (of, e.g., a detergent) can migrate to other acidic or anionic sites of other molecules. The products formed thereby, including the products formed upon employing the composition of the present invention in its intended use, may not be susceptible of easy description. Nevertheless, all such modifications and reaction products are included within the scope of the present invention; the present invention encompasses the composition prepared by admixing the components described above.

### EXAMPLES

The invention will be further illustrated by the following examples, which sets forth particularly advantageous embodiments. While the examples are provided to illustrate the present invention, they are not intended to limit it. Unless otherwise specified weight percents (wt. %) are given in wt. % based on the weight of the total composition. All results provided in Example Tables are an average value of at least three trials.

### Test Methods

### Viscosity

Brookfield rotating spindle method (all viscosity measurements reported herein are conducted by the Brookfield method whether mentioned or not): The viscosity measurements are calculated in centoise (cPs), employing a Brookfield rotating spindle viscometer, Model RVT (Brookfield Engineering Laboratories, Inc.), at about 20 revolutions per minute (rpm), at ambient room temperature of about 20 to 25°C (hereafter referred to as viscosity). Spindle sizes are selected in accordance with the standard operating recommendations from the manufacturer. Generally, spindle sizes are selected as follows:

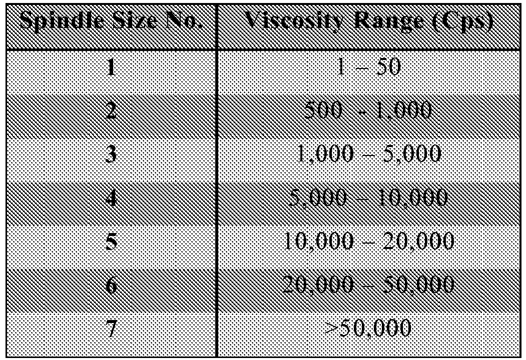

The spindle size recommendations are for illustrative purposes only. The artisan of ordinary skill in the art will select a spindle size appropriate for the system to be measured.

### Materials

The materials are generally commercially available from chemical supply houses known to those skilled in the chemical arts or from the supplier indicated below.

| | |
|---|---|
| Carbopol® 981NF | Carbomer homopolymer Type A available from The Lubrizol Corporation |
| Carbopol® 980NF | Carbomer homopolymer Type C available from The Lubrizol Corporation |
| Carbopol® ETD-2020-NF | Carbomer interpolymer Type B available from The Lubrizol Corporation |
| Pemulen™ TR2 NF | Carbomer copolymer Type A available from The Lubrizol Corporation |
| Noveon® AA-1 Polycarbophil, USP | Polycarbophil available from The Lubrizol Corporation |
| Euxyl-PE9010 | liquid preservative based on phenoxyethanol and ethylhexylglycerin available from Schülke, Inc. |
| Polyurethane A | ether based thermoplastic polyurethane from The Lubri zol Corporation |
| Polyurethane B | ether based thermoplastic polyurethane from The Lubri zol Corporation |
| THF | Tetrahydrofuran, ACS grade (99+%) stabilized with 250 ppm BHT available from Alfa Aesar |
| TRO | Tris(hydroxymethyl)aminomethane, ACS grade (99.8-100.1% assay, dried basis available from Alfa Aesar |
| TPU1 | aliphatic polyether thermoplastic polyurethane availa ble from The Lubrizol Corporation |
| TPU2 | hydrophilic polyether water-soluble thermoplastic polyurethane |
| TPU3 | highly water swellable aliphatic polyether thermo plastic polyurethane available from the Lubrizol Corpo ration |
| TPU4 | water swellable aliphatic polyether polyurethane |
| Ibuprofen, USP | Spectrum Chemical MFG. CORP., Gardena, CA |
| Metronidazole, USP | Medisco Inc., Plattsburgh, NY |
| Lidocaine, USP | Spectrum Chemical MFG. CORP., Gardena, CA |
| Lidocaine Hydrochloride, USP | Spectrum Chemical MFG. CORP., Gardena, CA |

### Example 1 - Preparation of blends of Carbopol® 981 NF polymer and TPU1 to be used for film casting

### Preparation of 1% aqueous Carbopol® 981 NF polymer dispersion

Using a mixer with a three blade marine impeller, 10 g of Carbopol® 981 NF polymer is dispersed to 990 g of deionized water according to procedures known in the art. The resulting dispersion is covered and let to rest overnight at room temperature.

### Neutralization of Carbopol® 981 NF polymer dispersion

The Carbopol® 981 NF polymer dispersion is partially neutralized with Tromethamine (30% aqueous solution) according to Table 1. The neutralizer is added slowly under continuous stirring. The pH and viscosity of the neutralized Carbopol® 981 NF polymer dispersion are measured after the dispersion is left to stand overnight at room temperature.

**Table 1**

| Amount of 1% aqueous Carbopol® 981NF dispersion (g) | Amount of Tromethamine 30% (w/w) aq (g) | pH of partially neutralized Carbopol® 981NF dispersion | Brookfiled viscosity* of partially neutralized Carbopol® 981NF dispersion (cP) |
|---|---|---|---|
| 1000 | 11 | 4.5 | 10600 |
| 1000 | 22 | 5.5 | 12140 |

| | | | |
|---|---|---|---|
| * Brookfield DV-I + Viscometer; 20 rpm | | | |

### Preparation of 3% (w/w) solution of TPU1 in THF/H₂O 90/10 (w/w)

Thirty (30) g of TPU1 is added to a 970 g mixture of THF/H₂O 90/10 (w/w) in a glass jar with lid. The mixture is shaken until dissolved at room temperature using a ThermoScientific MaxQ4000 orbital shaker.

### Preparation of blends of Carbopol® 981 NF polymer and TPU1 to be used for film casting

Several blends of Carbopol® 981 NF and TPU1 are prepared by mixing at room temperature the partially neutralized Carbopol® 981 NF polymer dispersion and the TPU1 according to Table 2. The viscosity and the pH of the resulting blends are listed in Table 3.

**Table 2**

| Example | Partially neutralized Carbopol® 981NF dispersion pH 4.5 (g) | Partially neutralized Carbopol® 981NF dispersion pH 5.5 (g) | 3% TPU1 (g) | Carbopol® 981NF : TPU1 |
|---|---|---|---|---|
| INV EX1 | 250 | - | 250 | 1:3 |
| INV EX2 | - | 250 | 250 | 1:3 |

**Table 3**

| Example | Brookfield viscosity* (cP) | pH |
|---|---|---|
| INV EX1 | 8650 | 5.82 |
| INV EX2 | 13200 | 6.22 |

| | | |
|---|---|---|
| * Brookfield DV-I + Viscometer; 20 rpm | | |

### Example 2 - Preparation of solvent cast films from Carbopol® 981 NF and TPU1 blends prepared in Example 1

Prior to film casting, air bubbles are removed from the blends prepared in Example 1 by centrifugation for 30 min at 1500 rpm using a Thermo Electron Corporation, IEC Centra GP8 Centrifuge. Degassed blends are used to solvent cast films on polyethylene substrates (5 mil natural high density polyethylene sheets from Griff Paper and Films, PA, USA) using an automatic film applicator with vacuum plate (Byko-drive with vacuum plate, Material # 2121; BYK Gardner, MD, USA), at a drawdown speed of 1 in/sec using a 100 mil gap clearance draw bar. Resulting films are dried in a laboratory hood at room temperature, in air. Drawdowns of various thicknesses can be prepared by adjusting the gap clearance of the draw bar.

### Example 3 - Film testing

### Film Fluid Absorption

A 5 cm x 5 cm film is weighed before placing it in a Petri dish. A volume of test solution A (Na⁺/Ca²⁺ 142mmol/L//2.5 mmol/L - aqueous solution), warmed up to 37 °C, equal to 40 times the weight of the test sample is dispensed slowly and gently onto the specimen in the Petri dish. The Petri dish and its content are then incubated at 37°C for 30 minutes in a convection oven with temperature control (Symphony™, VWR, USA). After 30 min, the dish is taken out of the oven. Using a pair of tweezers, the sample is removed from the Petri dish, suspended to allow excess solution A to drip off for 30 seconds and reweighed.
The test is repeated for 3 samples from the same lot. Results are expressed as both grams fluid absorbed per 10 cm² of film and grams fluid absorbed/g of film.
Table 4 lists the results for absorptive capacity of films prepared from INV EX1 and INV EX2 blends in Example 1.

### Dispersion characteristics/Wet integrity

A 5 cm x 5 cm film is placed into a 250 mL conical flask, to which is added 50 mL of solution A (Na⁺/Ca²⁺ 142mmol/L//2.5 mmol/L - aqueous solution) at 37°C. The flask is gently swirled for 60 seconds without causing a vortex, and the integrity of the dressing is visually established. Table 4 lists the results for dispersion characteristics/wet integrity for films prepared from the Carbopol® 981 NF: TPU1 blends in Example 1.

**Table 4**

| Example | Film Fluid Absorption | | Wet integrity |
|---|---|---|---|
| | g fluid absorbed/g film | g fluid absorbed/10 cm² | |
| INV EX1 | 5.49 | 0.27 | maintains integrity |
| INV EX2 | 7.92 | 0.47 | maintains integrity |

### Mechanical properties of dry films

Film samples for mechanical testing are analyzed according to ASTM D882-12. Prior to testing, film samples are conditioned at 23 ± 2 °C and 50 ± 5% relative humidity for no less than 40 hours.

A film strip for analysis has a width of approximately 6 mm and a thickness of 0.03 - 0.04 mm. The grip separation length is 50 mm and the crosshead speed is about 500 mm/min. To test if film properties change with direction, for each film, samples are cut parallel to film drawing direction (vertical) and perpendicular to film drawing direction (horizontal).

Samples were tested for Tensile Strength and % Elongation using a Texture Analyzer TA.XT*Plus* and Exponent Software (Texture Technologies, IL, USA).

For Young's Modulus measurement according to the ASTM D882-12 were done using an Instron 5564 instrument. Table 5 lists the results for mechanical properties for films prepared from the Carbopol® 981NF : TPU1 blends in Example 1.

**Table 5**

| Example | Dry films | | | |
|---|---|---|---|---|
| | Horizontal* | | Vertical** | |
| | Tensile strength (MPa) | %Elongation at break | Tensile strength (MPa) | %Elongation at break |
| INV EX1 | 23.31 | 316.32 | 21.64 | 300.49 |
| INV EX2 | 18.86 | 309.01 | 18.71 | 317.61 |

| | | | | |
|---|---|---|---|---|
| *Samples cut parallel with the film drawing direction **Samples cut perpendicular with the film drawing direction | | | | |

### Mechanical Properties of hydrated films

5 cm x 5 cm film samples are hydrated as described in the "Film Fluid Absorption" test. The hydrated samples are analyzed for mechanical properties such as burst strength, work to burst, distance to burst and stiffness by using a Texture Analyzer TA.XT*Plus* and Exponent Software (Texture Technologies, IL, USA). Table 6 lists the results for the mechanical properties for hydrated INV EX2 films of two different thicknesses.

**Table 6**

| INV FILM | Dry film thickness mil | Burst strength g | Work to burst g x mm | Distance to burst mm | Stiffness g/mm |
|---|---|---|---|---|---|
| INV EX2 | 1.57 | 69.78 | 589.07 | 19.28 | 4.06 |
| | 3.94 | 94.29 | 636.06 | 15.43 | 6.86 |

| | | | | | |
|---|---|---|---|---|---|
| Note: Films were hydrated in solution A for 30 min @ 37 °C prior to testing | | | | | |

### Moisture Vapor Transmission Rate (MVTR)

MVTR measurements are done using a Mocon Permatran-W® 101K per INDA WSP 70.4 (previously ASTM D6701) at 38 °C, 90% RH.

Films are peeled from the polyethylene substrate cut to fit the Mocon instrument cell and mounted in the instrument. Table 7 lists the results for MVTR for films prepared from blends in Example 1.

**Table 7**

| Example | Film Thickness (mil) | Moisture Vapor Transmission Rate g/[m²·day] |
|---|---|---|
| INV EX1 | 1.5 | 4073.99 |
| INV EX2 | 1.5 | 4668.45 |

### Example 4 - Preparation of blends of Carbopol® 981NF polymer and TPU2 to be used for film casting and preparation of Carbopol® 981NF polymer/TPU2 films

### Preparation of 1% aqueous Carbopol® 981NF polymer dispersion

Using a mixer with a three blade marine impeller, 10 g of Carbopol® 981NF polymer is dispersed to 990 g of deionized water according to procedures known in the art. The resulting dispersion is covered and let to rest overnight at room temperature.

### Neutralization of Carbopol® 981NF polymer dispersion

The Carbopol® 981NF polymer dispersion is partially neutralized with Tromethamine (30% aqueous solution) according to Table 1. The neutralizer is added slowly under continuous stirring. The pH and viscosity of the neutralized Carbopol® 981NF polymer dispersion are measured after the dispersion is left to stand overnight at room temperature.

### Preparation of 3% (w/w) solution of TPU2 in THF/H₂O 90/10 (w/w)

Thirty (30) g of TPU2 is added to a 970 g mixture of THF/H₂O 90/10 (w/w) in a glass jar with lid. The mixture is shaken until dissolved at room temperature using a ThermoScientific MaxQ4000 orbital shaker.

### Preparation of blends of Carbopol® 981NF polymer and TPU2 to be used for film casting

Several blends of Carbopol® 981NF and TPU2 are prepared by mixing at room temperature the partially neutralized Carbopol® 981NF polymer dispersion and the TPU2 according to Table 8. The viscosity and the pH of the resulting blends are listed in Table 9.

**Table 8**

| Example | Partially neutralized Carbopol® 981NF dispersion pH 4.5 (g) | Partially neutralized Carbopol® 981NF dispersion pH 5.5 (g) | 3% TPU2 (g) | Carbopol® 981NF : TPU2 |
|---|---|---|---|---|
| INV EX3 | 250 | - | 250 | 1:3 |
| INV EX4 | - | 250 | 250 | 1:3 |

**Table 9**

| Example | Brookfield viscosity* (cP) | pH |
|---|---|---|
| INV EX3 | 13900 | 5.90 |
| INV EX4 | 13850 | 6.25 |

| | | |
|---|---|---|
| * Brookfield DV-I + Viscometer; 20 rpm | | |

### Preparation of solvent cast films from Carbopol® 981 and TPU2 blends

Films of Carbopol® 981/TPU2 blends of composition indicated in Table 8 are prepared following the method described in Example 2.

### Example 5 - Characterization of films prepared in Example 4

Properties of films prepared in Example 4 are analyzed using the methods described in Example 3. Tables 10, 11 and 12 are illustrating the properties of films prepared in Example 4.

**Table 10**

| Example | Film Fluid Absorption | | Wet integrity |
|---|---|---|---|
| | g fluid absorbed/g film | g fluid absorbed/10 cm² | |
| INV EX3 | 16.04 | 0.78 | weak |
| INV EX4 | 27.04 | 1.38 | weak |

**Table 11**

| Example | Dry films | | | |
|---|---|---|---|---|
| | Horizontal* | | Vertical** | |
| | Tensile strength (MPa) | %Elongation at break | Tensile strength (MPa) | %Elongation at break |
| INV EX3 | 18.82 | 447 | 21.89 | 459 |
| INV EX4 | 22.95 | 367 | 21.34 | 367 |

| | | | | |
|---|---|---|---|---|
| *Samples cut parallel with the film drawing direction **Samples cut perpendicular with the film drawing direction | | | | |

**Table 12**

| Material | Film Thickness (mil) | Moisture Vapor Transmission Rate g/[m²·day] |
|---|---|---|
| INV EX3 | 1.7 | 5825.00 |
| INV EX4 | 2.0 | 3610.43 |

### Example 6 - Preparation of blends of Carbopol® 980NF polymer and TPU1 to be used for film casting and preparation of Carbopol® 980NF polymer/TPU1 films

### Preparation of 1% aqueous Carbopol® 980NF polymer dispersion

Using a mixer with a three blade marine impeller, 10 g of Carbopol® 980NF polymer is dispersed to 990 g of deionized water according to procedures known in the art. The resulting dispersion is covered and let to rest overnight at room temperature.

### Neutralization of Carbopol® 980NF polymer dispersion

The Carbopol® 980NF polymer dispersion is partially neutralized with Tromethamine (30% aqueous solution) according to Table 13. The neutralizer is added slowly under continuous stirring. The pH and viscosity of the neutralized Carbopol® 980NF polymer dispersion is measured after the dispersion is left to stand overnight at room temperature.

**Table 13**

| Amount of 1% aqueous Carbopol® 980NF dispersion (g) | Amount of Tromethamine 30% (w/w) aq (g) | pH of partially neutralized Carbopol® 980NF dispersion | Brookfiled viscosity* of partially neutralized Carbopol® 980NF dispersion (cP) |
|---|---|---|---|
| 1000 | 11 | 5.08 | 62800 |
| 1000 | 22 | 5.24 | 74200 |

| | | | |
|---|---|---|---|
| * Brookfield DV-I + Viscometer; 20 rpm | | | |

### Preparation of 3% (w/w) solution of TPU1 in THF/H₂O 90/10 (w/w)

Thirty (30) g of TPU1 is added to a 970 g mixture of THF/H₂O 90/10 (w/w) in a glass jar with lid. The mixture is shaken until dissolved at room temperature using a ThermoScientific MaxQ4000 orbital shaker.

### Preparation of blends of Carbopol® 980NF polymer and TPU1 to be used for film casting

Several blends of Carbopol® 980NF and TPU1 are prepared by mixing at room temperature the partially neutralized Carbopol® 980NF polymer dispersion and the TPU1 according to Table 14. The viscosity and the pH of the resulting blends are listed in Table 15.

**Table 14**

| Example | Partially neutralized Carbopol® 980NF dispersion pH 5.08 (g) | Partially neutralized Carbopol® 980NF dispersion pH 5.24 (g) | 3% TPU1 (g) | Carbopol® 980NF : TPU1 |
|---|---|---|---|---|
| INV EX5 | 250 | - | 250 | 1:3 |
| INV EX6 | - | 250 | 250 | 1:3 |

**Table 15**

| Example | Brookfield viscosity* (cP) | pH |
|---|---|---|
| INV EX5 | 44000 | 6.29 |
| INV EX6 | 47000 | 6.68 |

| | | |
|---|---|---|
| * Brookfield DV-I + Viscometer; 20 rpm | | |

### Example 7 - Preparation of blends of Pemulen™TR2 NF polymer and TPU1 to be used for film casting

### Preparation of 1% aqueous Pemulen™TR2 NF polymer dispersion

Using a mixer with a three blade marine impeller, 10 g of Pemulen™TR2 NF polymer is dispersed to 990 g of deionized water according to procedures known in the art. The resulting dispersion is covered and let to rest overnight at room temperature.

### Neutralization of Pemulen™TR2 NF polymer dispersion

The Pemulen™TR2 NF polymer dispersion is partially neutralized with Tromethamine (30% aqueous solution) according to Table 16. The neutralizer is added slowly under continuous stirring. The pH and viscosity of the neutralized Pemulen™TR2 NF polymer dispersion are measured after the dispersion is left to stand overnight at room temperature.

**Table 16**

| Amount of 1% aqueous Pemulen™ TR2 NF dispersion (g) | Amount of Tromethamine 30% (w/w) aq (g) | pH of partially neutralized Pemulen™TR2 NF dispersion | Brookfiled viscosity* of partially neutralized Pemulen™TR2 NF dispersion (cP) |
|---|---|---|---|
| 1000 | 11 | 4.55 | 22,500 |

| | | | |
|---|---|---|---|
| * Brookfield DV-I + Viscometer; 20 rpm | | | |

### Preparation of 3% (w/w) solution of TPU1 in THF/H₂O 90/10 (w/w)

Thirty (30) g of TPU1 is added to a 970 g mixture of THF/H₂O 90/10 (w/w) in a glass jar with lid. The mixture is shaken until dissolved at room temperature using a ThermoScientific MaxQ4000 orbital shaker.

### Preparation of blends of Pemulen™TR2 NF polymer and TPU1 to be used for film casting

Several blends of Pemulen™TR2 NF and TPU1 are prepared by mixing at room temperature the partially neutralized Pemulen™TR2 NF polymer dispersion and the TPU1 according to Table 17. The viscosity and the pH of the resulting blends are listed in Table 18.

**Table 17**

| Example | Partially neutralized Pemulen™TR2 NF dispersion pH 4.5 (g) | 3% TPU1 (g) | Pemulen™ TR2 NF: TPU1 |
|---|---|---|---|
| INV EXP 1 | 250 | 250 | 1:3 |

**Table 18**

| Example | Brookfield viscosity* (cP) | pH |
|---|---|---|
| INV EXP1 | 4500 | 5.90 |

| | | |
|---|---|---|
| * Brookfield DV-I + Viscometer; 20 rpm | | |

### Preparation of solvent cast films from Pemulen™ TR2 and TPU1 blends

Films of Pemulen™TR2 NF/TPU1 blends of composition indicated in Table 17 are prepared following the method described in Example 2.

### Example 8 - Characterization of films prepared in Example 7

Properties of films prepared in Example 7 are analyzed using the methods described in Example 3. Tables 19, 20, 21 and 22 are illustrating the properties of films prepared in Example 7.

**Table 19**

| Example | Film Fluid Absorption | | Wet integrity |
|---|---|---|---|
| | g fluid absorbed/g film | g fluid absorbed/10 cm² | |
| INV EXP1 | 4.9 | 0.28 | Very good |

**Table 20**

| Example | Dry films | | | |
|---|---|---|---|---|
| | Horizontal* | | Vertical** | |
| | Tensile strength (MPa) | %Elongation at break | Tensile strength (MPa) | %Elongation at break |
| INV EXP1 | 12.41 | 357.03 | 11.57 | 331.32 |

| | | | | |
|---|---|---|---|---|
| *Samples cut parallel with the film drawing direction **Samples cut perpendicular with the film drawing direction | | | | |

**Table 21**

| | Burst strength g | Work to burst g x mm | Distance to burst mm | Stiffness g/mm |
|---|---|---|---|---|
| INV EXP1 1 | 226.67 | 2542.6 | 30.99 | 8.84 |

| | | | | |
|---|---|---|---|---|
| Note: Films were hydrated in solution A for 30 min @ 37 °C prior to testing | | | | |

**Table 22**

| Material | Film Thickness (mil) | Moisture Vapor Transmission Rate g/[m²·day] |
|---|---|---|
| INV EXP1 | 1.8 | 5634.95 |

### Example 9 - Preparation of blends of Noveon® AA-1 USP Polycarbophil polymer and TPU1 to be used for film casting

### Preparation of 1% aqueous Noveon® AA-1 USP Polycarbophil polymer dispersion

Using a mixer with a three blade marine impeller, 10 g of Noveon® AA-1 USP Polycarbophil polymer is dispersed to 990 g of deionized water according to procedures known in the art. The resulting dispersion is covered and let to rest overnight at room temperature.

### Neutralization of Noveon® AA-1 USP Polycarbophil polymer dispersion

The Noveon® AA-1 USP Polycarbophil polymer dispersion is partially neutralized with Tromethamine (30% aqueous solution) according to Table 23. The neutralizer is added slowly under continuous stirring. The pH and viscosity of the neutralized Noveon® AA-1 USP Polycarbophil polymer dispersion are measured after the dispersion is left to stand overnight at room temperature.

**Table 23**

| Amount of 1% aqueous Noveon® AA-1 USP Polycarbophil dispersion (g) | Amount of Tromethamine 30% (w/w) aq (g) | pH of partially neutralized Noveon® AA-1 USP Polycarbophil dispersion | Brookfiled viscosity* of partially neutralized Noveon® AA-1 USP Polycarbophil dispersion (cP) |
|---|---|---|---|
| 1000 | 11 | 4.6 | 21100 |
| 1000 | 22 | 5.7 | 24500 |

| | | | |
|---|---|---|---|
| * Brookfield DV-I + Viscometer; 20 rpm | | | |

### Preparation of 3% (w/w) solution of TPU1 in THF/H₂O 90/10 (w/w)

Thirty (30) g of TPU1 is added to a 970 g mixture of THF/H₂O 90/10 (w/w) in a glass jar with lid. The mixture is shaken until dissolved at room temperature using a ThermoScientific MaxQ4000 orbital shaker.

### Preparation of blends of Noveon® AA-1 USP Polycarbophil polymer and TPU1 to be used for film casting

Several blends of Noveon® AA-1 USP Polycarbophil and TPU are prepared by mixing at room temperature the partially neutralized Noveon® AA-1 USP Polycarbophil polymer dispersion and the TPU according to Table 24. The viscosity and the pH of the resulting blends are listed in Table 25.

**Table 24**

| Example | Partially neutralized Noveon® AA-1 USP Polycarbophil dispersion pH 4.6 (g) | Partially neutralized Noveon® AA-1 USP Polycarbophil dispersion pH 5.7 (g) | 3% TPU1 (g) | Noveon® AA-1 USP Polycarbophil : TPU-1 |
|---|---|---|---|---|
| INV EXPC1 | 250 | - | 250 | 1:3 |
| INV EXPC2 | - | 250 | 250 | 1:3 |

**Table 25**

| Example | Brookfield viscosity* (cP) | pH |
|---|---|---|
| INV EXPC1 | 15000 | 6.24 |
| INV EXPC2 | 17100 | 6.66 |

| | | |
|---|---|---|
| * Brookfield DV-I + Viscometer; 20 rpm | | |

### Preparation of solvent cast films from Noveon® AA-1 USP Polycarbophil and TPU1 blends

Films of Noveon® AA-1 USP Polycarbophil/TPU1 blends of composition indicated in Table 24 are prepared following the method described in Example 2.

### Example 10 - Characterization of films prepared in Example 9

Properties of films prepared in Example 9 are analyzed using the methods described in Example 3. Tables 26, 27 and 28 are illustrating the properties of films prepared in Example 9.

**Table 26**

| Example | Film Fluid Absorption | | Wet integrity |
|---|---|---|---|
| | g fluid absorbed/g film | g fluid absorbed/10 cm² | |
| INV EXPC1 | 5.32 | 2.13 | Good |
| INV EXPC2 | 7.84 | 3.13 | Good |

**Table 27**

| | Tensile strength (MPa) | %Elongation at break |
|---|---|---|
| INV EXPC1 | 9.92 | 283.12 |
| INV EXPC2 | 7.93 | 339.84 |

**Table 28**

| | Burst strength g | Work to burst g x mm | Distance to burst mm | Stiffness g/mm |
|---|---|---|---|---|
| INV EXPC1 | 175.24 | 1154.84 | 17.70 | 12.17 |
| INV EXPC2 | 79.58 | 638.77 | 18.65 | 5.05 |

### Example 11 - Preparation of blends of Carbopol® 981NF/Pemulen™TR2 NF (1/1 w/w) polymer and TPU1 to be used for film casting

### Preparation of 1% aqueous Carbopol® 981NF/Pemulen™TR2 NF (1/1 w/w) polymer dispersion

Using a mixer with a three blade marine impeller, 3.25 g of Carbopol® 981NF and 3.25 g Pemulen™TR2 NF polymer are dispersed to 643.5 g of deionized water according to procedures known in the art. The resulting dispersion is covered and let to rest overnight at room temperature.

### Neutralization of 1% aqueous Carbopol® 981NF/Pemulen™TR2 NF (1/1 w/w) polymer dispersion

The 1% aqueous Carbopol® 981NF/Pemulen™TR2 NF (1/1 w/w) polymer dispersion is partially neutralized with Tromethamine (30% aqueous solution) according to Table 29. The neutralizer is added slowly under continuous stirring. The pH and viscosity of the neutralized Carbopol® 981NF/Pemulen™TR2 NF (1/1 w/w) polymer dispersion are measured after the dispersion is left to stand overnight at room temperature.

**Table 29**

| Amount of 1% aqueous Carbopol® 981NF/Pemulen™ TR2 NF (1/1 w/w) dispersion (g) | Amount of Tromethamine 30% (w/w) aq (g) | pH of partially neutralized 1% aqueous Carbopol® 981NF/Pemulen™ TR2 NF (1/1 w/w) dispersion | Brookfiled viscosity* of partially neutralized 1% aqueous Carbopol® 981NF/Pemulen™T R2 NF (1/1 w/w) dispersion (cP) |
|---|---|---|---|
| 650 | 5 | 4.56 | 13180 |
| 650 | 11 | 5.45 | 14760 |

| | | | |
|---|---|---|---|
| * Brookfield DV-I + Viscometer; 20 rpm | | | |

### Preparation of 3% (w/w) solution of TPU1 in THF/H₂O 90/10 (w/w)

19.5 g of TPU1 is added to a 630.5 g mixture of THF/H₂O 90/10 (w/w) in a glass jar with lid. The mixture is shaken until the polymer is dissolved at room temperature using a ThermoScientific MaxQ4000 orbital shaker.

### Preparation of blends of 1% aqueous Carbopol® 981NF/Pemulen™TR2 NF (1/1 w/w) polymer dispersion and TPU-1 to be used for film casting

Several blends of 1% aqueous Carbopol® 981NF/Pemulen™TR2 NF (1/1 w/w) partially neutralized dispersion and TPU1 are prepared by mixing at room temperature the partially neutralized 1% aqueous Carbopol® 981NF/Pemulen™TR2 NF (1/1 w/w) dispersion and the TPU1 according to Table 30. The viscosity and the pH of the resulting blends are listed in Table 31.

**Table 30**

| Example | Partially neutralized 1% aqueous Carbopol® 981NF/Pemul en™TR2 NF (1/1 w/w) dispersion pH 4.5 (g) | Partially neutralized 1% aqueous Carbopol® 981NF/Pemulen ™TR2 NF (1/1 w/w) dispersion pH 5.5 (g) | 3% TPU1 (g) | Carbopol® 981NF/Pemulen™ TR2 NF (1/1 w/w) : TPU1 |
|---|---|---|---|---|
| INV EXCP1 | 250 | - | 250 | 1:3 |
| INV EXCP2 | - | 250 | 250 | 1:3 |

**Table 31**

| Example | Brookfield viscosity* (cP) | pH |
|---|---|---|
| INV EXCP1 | 7250 | 6.21 |
| INV EXCP2 | 7335 | 6.53 |

| | | |
|---|---|---|
| * Brookfield DV-I + Viscometer; 20 rpm | | |

### Preparation of solvent cast films from 1% aqueous Carbopol® 981NF/Pemulen™TR2 NF (1/1 w/w)/TPU1 blends

Films of Carbopol® 981NF/Pemulen™TR2 NF (1/1 w/w)/TPU1 blends of composition indicated in Table 30 are prepared following the method described in Example 2.

### Example 12 - Characterization of films prepared in Example 11

Properties of films prepared in Example 11 are analyzed using the methods described in Example 3. Tables 32, 33, 34 and 35 are illustrating the properties of films prepared in Example 11.

**Table 32**

| Example | Film Fluid Absorption | | Wet integrity |
|---|---|---|---|
| | g fluid absorbed/g film | g fluid absorbed/10 cm² | |
| INV EXCP1 | 4.70 | 1.88 | Very good |
| INV EXCP2 | 6.42 | 2.57 | Very good |

**Table 33**

| | Tensile strength (MPa) | %Elongation at break |
|---|---|---|
| INV EXCP1 | 11.21 | 554.45 |
| INV EXCP2 | 11.37 | 419.25 |

**Table 34**

| | Burst strength g | Work to burst g x mm | Distance to burst mm | Stiffness g/mm |
|---|---|---|---|---|
| INV EXCP1 | 106.05 | 1141.50 | 22.80 | 5.70 |
| INV EXCP2 | 81.25 | 771.75 | 19.6 | 4.5 |

| | | | | |
|---|---|---|---|---|
| Note: Films were hydrated in solution A for 30 min @ 37 °C prior to testing | | | | |

**Table 35**

| Material | Film Thickness (mil) | Moisture Vapor Transmission Rate g/[m²·day] |
|---|---|---|
| INV EXCP1 | 1.5 | 4031.05 |
| INVEXCP2 | 1.5 | 4818.88 |

### Example 13 Preparation of blends of Carbopol® 981NF/Pemulen™TR2 NF (1/2 w/w) polymer and TPU1 to be used for film casting

### Preparation of 1% aqueous Carbopol® 981NF/Pemulen™TR2 NF (1/2 w/w) polymer dispersion

Using a mixer with a three blade marine impeller, 2.17 g of Carbopol® 981NF and 4.33 g Pemulen™ TR2 polymer are dispersed to 643.5 g of deionized water according to procedures known in the art. The resulting dispersion is covered and let to rest overnight at room temperature.

### Neutralization of 1% aqueous Carbopol® 981NF/Pemulen™TR2 NF (1/2 w/w) polymer dispersion

The 1% aqueous Carbopol® 981NF/Pemulen™TR2 NF (1/2 w/w) polymer dispersion is partially neutralized with Tromethamine (30% aqueous solution) according to Table 36. The neutralizer is added slowly under continuous stirring. The pH and viscosity of the neutralized Carbopol® 981NF/Pemulen™TR2 NF (1/2 w/w) polymer dispersion are measured after the dispersion is left to stand overnight at room temperature.

**Table 36**

| Amount of 1% aqueous Carbopol®981NF/ Pemulen™TR2 NF(1/2 w/w) dispersion (g) | Amount of Tromethamine 30% (w/w) aq (g) | pH of partially neutralized 1% aqueous Carbopol® 981NF/Pemulen™ TR2 NF (1/2 w/w) dispersion | Brookfiled viscosity* of partially neutralized 1% aqueous Carbopol® 981NF/Pemulen™TR2 (1/2 w/w) dispersion (cP) |
|---|---|---|---|
| 650 | 5.5 | 4.50 | 13000 |
| 650 | 11.5 | 5.39 | 13700 |

| | | | |
|---|---|---|---|
| * Brookfield DV-I + Viscometer; 20 rpm | | | |

### Preparation of 3% (w/w) solution of TPU1 in THF/H₂O 90/10 (w/w)

19.5 g of TPU1 is added to a 630.5 g mixture of THF/H₂O 90/10 (w/w) in a glass jar with lid. The mixture is shaken until the polymer is dissolved at room temperature using a ThermoScientific MaxQ4000 orbital shaker.

### Preparation of blends of 1% aqueous Carbopol® 981NF/Pemulen™TR2 NF (1/2 w/w) polymer dispersion and TPU1 to be used for film casting

Several blends of 1% aqueous Carbopol® 981NF/Pemulen™TR2 NF (1/2 w/w) partially neutralized dispersion and TPU1 are prepared by mixing at room temperature the partially neutralized 1% aqueous Carbopol® 981NF/Pemulen™TR2 NF (1/2 w/w) dispersion and the TPU1 according to Table 37. The viscosity and the pH of the resulting blends are listed in Table 38.

**Table 37**

| Example | Partially neutralized 1% aqueous Carbopol®981NF/ Pemulen™TR2 NF (1/2 w/w) dispersion pH 4.5 (g) | Partially neutralized 1% aqueous Carbopol® 981NF/ Pemulen™TR2 NF (1/2 w/w) dispersion pH 5.5 (g) | 3% TPU1 (g) | Carbopol®98 1NF/ Pemulen™T R2 NF (1/2 w/w) : TPU1 |
|---|---|---|---|---|
| INV EXCP3 | 250 | - | 250 | 1:3 |
| INV EXCP4 | - | 250 | 250 | 1:3 |

**Table 38**

| Example | Brookfield viscosity* (cP) | pH |
|---|---|---|
| INV EXCP3 | 6300 | 6.20 |
| INV EXCP4 | 6500 | 6.64 |

| | | |
|---|---|---|
| * Brookfield DV-I + Viscometer; 20 rpm | | |

### Preparation of solvent cast films from 1% aqueous Carbopol® 981NF/Pemulen™TR2 NF (1/2 w/w)/TPU1 blends

Films of Carbopol® 981NF/Pemulen™TR2 NF (1/2 w/w)/TPU1 blends of composition indicated in Table 37 are prepared following the method described in Example 2.

### Example 14 Characterization of Films prepared in Example 13

Properties of films prepared in Example 13 are analyzed using the methods described in Example 3. Tables 39, 40, 41 and 42 are illustrating the properties of films prepared in Example 13.

**Table 39**

| Example | Hydration duration | Film Fluid Absorption | | Wet integrity |
|---|---|---|---|---|
| | | g fluid absorbed/g film | g fluid absorbed/10 cm² | |
| INV EXCP3 | 30 min | 4.26 | 1.70 | Very good |
| | 24 h | 4.30 | 1.72 | Very good |
| INV EXCP4 | 30 min | 4.35 | 1.74 | Very good |
| | 24 h | 5.58 | 2.23 | Very good |

**Table 40**

| | Tensile strength (MPa) | %Elongation at break |
|---|---|---|
| INV EXCP3 | 12.18 | 566.2 |
| INV EXCP4 | 10.27 | 395.37 |

**Table 41**

| | Hydration duration before test | Burst strength g | Work to burst gxmm | Distance to burst mm | Stiffness g/mm |
|---|---|---|---|---|---|
| INV EXCP3 | 30 min | 148.9 | 1852.7 | 26.25 | 7.05 |
| | 24 h | 158.36 | 2044.22 | 27.44 | 7.04 |
| INV EXCP4 | 30 min | 143.55 | 1348.25 | 21.2 | 7.95 |
| | 24 h | 129.39 | 1420.20 | 24.8 | 5.99 |

**Table 42**

| Material | Film Thickness (mil) | Moisture Vapor Transmission Rate g/[m²·day] |
|---|---|---|
| INV EXCP3 | 1.5 | 4295.63 |
| INV EXCP4 | 1.5 | 4953.37 |

### Example 15 - Preparation of bilayer films consisting of a polyurethane backing layer and an invention film layer

16% (w/w) Polyurethane A and 12% (w/w) Polyurethane B solutions in THF are prepared.

Polymer blends corresponding to INV EX2 and INV EXP1 are prepared according to the method described in Example 1 and Example 7, respectively. The resulted solutions and blends are degassed by centrifugation for 30 min at 1500 rpm using a Thermo Electron Corporation, IEC Centra GP8 Centrifuge.

The degassed blends are used to solvent cast films on polyethylene substrates (5 mil natural high density polyethylene sheets from Griff Paper Films, PA, USA) using an automatic film applicator with vaccum plate (Byko- drive with vacuum plate, material # 2121; BYK Gardner, MD, USA), at a drawdown speed of 1 in/sec. First layer to be cast is the polyurethane layer. The thickness in mil of the polyurethane drawdown (before solvent evaporation) is indicated in Table 43. The invention film is then cast immediately on the polyurethane layer. The thickness in mil of the invention film layer (before solvent evaporation) is indicated in Table 43. The resulted bilayer film drawdown is allowed to dry in air at room temperature. Those skilled in the art can easily envision that the polyethylene sheet can be used as a liner to support the bilayer films. Also order of layer casting can be changed such that the polyethylene substrate is in direct contact with the invention film.

**Table 43**

| Drawdown thickness (mil) for bilayer films prepared according to Example 15 | | | | |
|---|---|---|---|---|
| Example | Polyurethane A | Polyurethane B | INV EX1 | INV EXP 1 |
| INV EX Bilayer1 | 5 | - | 100 | - |
| INV EX Bilayer2 | - | 5 | 100 | - |
| INV EX Bilayer3 | 5 | - | - | 100 |
| INV EX Bilayer4 | - | 5 | - | 100 |
| INV EX Bilayer5 | - | 5 | - | 200 |

### Example 16 - Characterization of bilayer films prepared in Example 15

Film fluid absorption and MVTR of the films prepared in Example 15 are analyzed using the methods described in Example 3. Tables 44 and 45 are illustrating the properties of films prepared in Example 15.

**Table 44**

| Example | Film Fluid Absorption | | Wet integrity |
|---|---|---|---|
| | g fluid absorbed/g film | g fluid absorbed/10 cm² | |
| INV EX Bilayer1 | 5.42 | 0.45 | Very good |
| INV EX Bilayer2 | 5.25 | 0.36 | Very good |
| INV EX Bilayer3 | 2.54 | 0.18 | Very good |
| INV EX Bilayer4 | 2.12 | 0.19 | Very good |
| INV EX Bilayer5 | 2.31 | 0.30 | Very good |

**Table 45**

| Material | Dry Film Thickness (mil) | Moisture Vapor Transmission Rate g/[m²·day] |
|---|---|---|
| INV EX Bilayer1 | 2.5 | 4132.22 |
| INV EX Bilayer2 | 1.5 | 3790.31 |
| INV EX Bilayer3 | 2.5 | 3847.37 |
| INV EX Bilayer4 | 2.0 | 3299.57 |
| INV EX Bilayer5 | 4.0 | 2274.18 |

### Comparative Example 1

### Preparation and characterization of solvent cast films of TPU1, TPU2 and Polyurethane A (COMP EX1) and Polyurethane B (COMP EX2)

Solutions of TPU1, TPU2 and TPUs (COMP EX1 and COMPEX2) used in commercial wound dressings are prepared by adding the polymer to the solvent and then alternating mixing at room temperature and heating at 40 - 42 °C for periods of 30 min to 1 h, until the polymer is dissolved. Table 46 lists the concentration of the solutions prepared and the solvents used.

**Table 46**

| Material | Solvent | Concentration of solution prepared % (w/w) |
|---|---|---|
| TPU-1 | THF : H₂O 90 : 10 | 10 |
| TPU-2 | THF : H₂O 90 : 10 | 10 |
| COMP EX1* | THF | 17 |
| COMP EX2** | THF | 17 |

| | | |
|---|---|---|
| *Estane 58245-031 available from The Lubrizol Corporation **Estane 58237-024 available from The Lubrizol Corporation | | |

Films are cast from the solutions prepared in Table 46 using a 25 mil draw bar according to the procedure described in Example 2. The resulting dry films are characterized using the methods described in Example 3. Tables 47, 48, and 49 illustrate the values obtained for films prepared in Comparative Example 1 for film fluid absorption, mechanical properties and MVTR, respectively.

**Table 47**

| Material | Film Fluid Absorption | | Wet integrity |
|---|---|---|---|
| | g fluid absorbed/ g film | g fluid absorbed/ 10 cm² | |
| TPU1 | 4.11 | 0.25 | maintains integrity |
| TPU2 | not able to measure* | not able to measure* | very weak gel that dissolves in water |
| COMP EX1 | NO | NO | maintains integrity |
| COMP EX2 | NO | NO | maintains integrity |

| | | | |
|---|---|---|---|
| *TPU2 film in contact with *solution A* absorbs fluid and forms an extremely weak gel that disintegrates. | | | |

**Table 48**

| Material | Horizontal* | | Vertical** | |
|---|---|---|---|---|
| | Tensile Strength (MPa) | % Elongation at break | Tensile Strength (MPa) | % Elongation at break |
| TPU1 | N/A*** | N/A*** | N/A*** | N/A*** |
| TPU2 | 15.45 | 592 | 15.65 | 610 |
| COMP EX1 | 21.51 | 444 | 19.31 | 429 |
| COMP EX2 | 22.71 | 336 | 27.30 | 359 |

| | | | | |
|---|---|---|---|---|
| *Samples cut parallel with the film drawing direction; **Samples cut perpendicular with the film drawing direction; *** Film is hard to peel from the substrate and accurate measurements could not be made | | | | |

**Table 49**

| Material | Film Thickness (mil) | Moisture Vapor Transmission Rate g/[m²·day] |
|---|---|---|
| TPU1 | - | N/A |
| TPU2 | 1.5 | 6669.87 |
| COMP EX1 | 2.2 | 3434.59 |
| COMP EX2 | 2.2 | 1736.55 |

As can be seen from the results presented, the films prepared according to the present invention have a unique set of properties when compared with TPUs used in commercial products. The films prepared according to the present invention combine the fluid absorption characteristic of cross-linked poly(acrylic) acid polymers, the film forming properties of TPUs, and the mechanical properties of films formed by TPUs.

### Example 17 - Characterization of commercial wound dressing films based on polyurethane polymers

Commercial wound dressing films based on TPU polymers are characterized using methods described in Example 3. Mechanical properties and MVTR of these films are listed in Table 50 and Table 51, respectively.

**Table 50**

| Product*/Manufactu rer | Tensile strength (Horizontal) (MPa) | Tensile Strength (Vertical) (MPa) | %Elongati on (Horizonta l) | %Elongation (Vertical) |
|---|---|---|---|---|
| Tegaderm™ Film/ 3M | 28.49 | 26.95 | 466 | 478 |
| Hydrofilm®/ Hartmann | 37.4 | 38.65 | 470 | 484 |
| Mepore Mepore® Film/Mölnlycke Health Care | 24.59 | 22.25 | 395 | 375 |
| POLYSKIN™ II/Kendall | 15.79 | 14.7 | 470 | 484 |
| Opsite Flexigrid/ Smith&Nephew | 33.42 | 30.74 | 554 | 554 |

| | | | | |
|---|---|---|---|---|
| *These commercial wound dressings have an adhesive layer coated on the polymer film | | | | |

**Table 51**

| Product*/ Manufacturer | Thickness (mil) | Moisture Vapor Transmission Rate g/[m2•day] |
|---|---|---|
| Tegaderm™ Film/3M | 1.5 | 630.82 |
| POLYSKIN™ II/Kendall | 2.0 | 647.25 |
| Opsite Flexigrid/Smith&Nephew | 2.5 | 564.17 |
| Hydrofilm®/Hartmann | 1.5 | 1481.60 |
| Mepore®Film/ Mölnlycke Health Care | 2.0 | 1001.47 |

| | | |
|---|---|---|
| *These commercial wound dressings have an adhesive layer coated on the polymer film | | |

As illustrated in Example 17, polyurethane used in commercial products do not have fluid absorbent properties and films of TPU2 are not able to maintain integrity in a fluid environment. Moreover, some of the TPU polymers are not easy to remove from substrates and make handling difficult, thus impairing the accurate measurement of the properties.

### Comparative Example 2

75 g of TPU3 is dissolved in 425 g of a 80:20 ethanol:water to give a 15 wt% solution with a Brookfield viscosity of 4920 Cps. The solution is cast with an 8 inch draw down bar set at a thickness of 100 mil. The film is cast onto four separate release substrates: (i) High Density Polyethylene (HDPE) certified with no additives; (ii) Teflon; (iii) HDPE release film; and (iv) Low Density Polyethylene (LDPE). The films are allowed to dry in a hood overnight. In all cases, the films dried unevenly and popped up from the surface, giving a curly material unsuitable for use as a film.

### Comparative Example 3

90 g of TPU1 is dissolved in 510 g of 80 : 20 ethanol : water to give a 15 wt% solution with a Brookfield viscosity of 6,280 cPs. The solution is cast with an 8 in draw down bar set at a thickness of 100 mil. The film dried unevenly and popped up from the surface with uneven thickness.

### Example 18

A 1 wt% dispersion of Carbopol® ETD 2020 NF is made in a 50 : 50 ethanol : water solution. The polymer is neutralized to pH 5 with TRO and preserved with 0.2 wt% Euxyl-PE9010. TPU3 is dissolved in 80 : 20 ethanol : water solution at 15 wt%. The two solutions are combined at a ratio to give a final wt% of Carbopol® ETD 2020 NF of 0.2 wt% and TPU3 of 12 wt% and cast as a film with an 8 in draw down bar set at a thickness of 100 mil on a release substrate. The film dried to an average thickness of 8.7 mil and is listed as INV EX7. Table 52 gives the polymers and film information. The Carbopol® ETD 2020 NF solution described above and the TPU1 dissolved in 80 : 20 ethanol : water at 15 wt% are mixed. The two solutions are combined at a ratio to give a final wt% of Carbopol® ETD 2020 NF of 0.2 wt% and TPU1 of 12 wt%. The viscosity of the solution is 23,300 cPs and cast as a film with an 8 in draw down bar set at a thickness of 100 mil on a release substrate. The film dried to an average thickness of 8.27 mil. The film dried to a smooth film without wrinkles or curls and adhered to the HDPE release film and is listed as INV EX8.

A 1 wt% dispersion of Carbopol® 981NF is made in 50 : 50 ethanol: water solution. The polymer was neutralized to pH 5 with TRO and preserved with 0.2 wt% Euxyl-PE9010. TPU3 solution from above in 80 : 20 ethanol : water solution at 15 wt% is used. The two solutions were combined at ratio to give a final wt% of Carbopol® 981NF of 0.2 wt% and TPU3 of 12 wt%. The viscosity of the solution is 9,900 cPs and cast as a film with an 8 in draw down bar set at a thickness of 100 mil on release substrate. The film dried to a smooth film without wrinkles or curls with an average thickness of 7.1 mil and is listed as INV EX9 in Table 52.

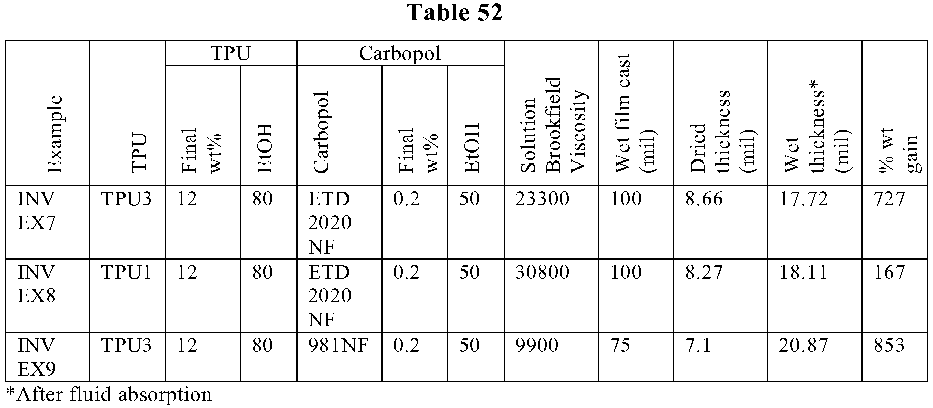

As apparent from the above Table 52, the addition of poly(acrylic) acid and poly(acrylic) acid interpolymer to TPU1 and TPU3 enhances the dry film properties by producing a smooth film of consistent thickness that may be easily removed from the casting substrate. Without the poly(acrylic) acid, the films are uneven or unable to be removed from the substrate without distortion, as seen in Comparative Examples 2 and 3.

### Example 19

6.6 g of Carbopol® ETD 2020 NF is dispersed in 100 g of THF and added to 100 g of water. The solution viscosity is 11,240 cPs. The solution is neutralized using ammonium hydroxide to pH 6.7 to give a gel of 32,200 cPs. A combination of 50 g TPU3 and 50 g TPU4 are dissolved in a 148 g THF and 148 g water solvent mixture. The Carbopol® ETD 2020 NF and TPU solutions are combined and mixed. The combined solution is cast into a mold and allowed to air dry into a film. Once the film is completely dried, it is removed from the mold, and a circular piece cut and soaked in distilled water to give water pickup and swell data presented in Table 53. This film is listed as INV EX10.

### Example 20

4 g of Carbopol® ETD 2020 NF is dispersed in 100 g THF and added to 100 g of water. The solution is neutralized to pH 6.3 using ammonium hydroxide to give a gel of 26,650 cPs. A combination of TPU3 50 g and TPU4 50 g are dissolved in 148 g THF and 148 g water solvent mixture. The Carbopol® ETD 2020 NF and TPU solutions are combined and mixed. The combined solution is cast into a mold and allowed to air dry into a film. Once the film is completely dried, it is removed from the mold, and a circular piece cut and soaked in distilled water to give the water pickup and swell data presented in Table 53. This film is listed as INV EX11.

### Example 21

4 g of Carbopol® ETD 2020 NF is dispersed in THF (100 g) and then water (100 g) is added. The solution is neutralized using ammonium hydroxide to pH 6.3 to give a gel of 26,650 cPs. A combination of TPU3 50 g and TPU4 50 g are dissolved in THF 360 g and H₂O 360 g solvent mixture. The Carbopol® ETD 2020 NF and TPU solutions are combined and mixed. The combined solution is cast into a mold and allowed to air dry into a film. Once the film is completely dried, it is removed from the mold, and a circular piece cut and soaked in distilled water to give the water pickup and swell data presented in Table 53. This film is listed as INV EX12.

### Comparative Example 4

A combination of 50 g TPU3 and 50 g TPU4 are dissolved in 148 g THF and 148 g water solvent mixture. The combined solution is cast into a mold and allowed to air dry. Once the film is completely dried, it is removed from the mold, and a circular piece cut and soaked in distilled water to give the water pickup and swell data presented in Table 53.

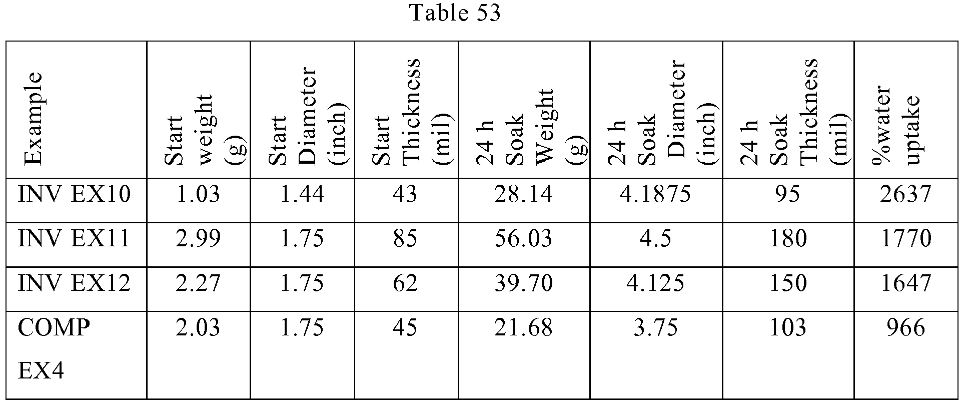

As can be seen from Table 53, the above examples illustrate the ability to prepare thick films that will hydrate to hydrogel films in DI (deionized) water. These hydrogel films have density close to water, good strength, and a low coefficient of friction that are suitable as acoustic transmission media.

### Example A - Preparation of Ibuprofen/Carbopol® 981 NF (pH 5.5)/TPU1 films and Ibuprofen release from the prepared films

To 500 g blend of Carbopol® 981 NF (pH 5.5) and TPU1 prepared according to Example 1 add under continuous stirring 5% Ibuprofen solution in THF according to Table a1. Let the resulted mixture stand overnight and measure viscosity and pH. From the mixture cast films using a 100 mil and 200 mil gap clearance bar, respectively, according to Example 2. The films resulted from the compositions described herein will constitute INV EXA1, INV EXA2 and INV EXA3. Table Aa illustrates the amount of Ibuprofen solution added and the pH of the Ibuprofen containing blend that are used to cast INV EXA1, INV EXA2 and INV EXA3, respectively.

**Table Aa**

| Example | Amount of Carbopol® 981 NF (pH 5.5)/TPU1 blend (g) | Amount of 5% IBU in THF (g) | pH of resulted mixture |
|---|---|---|---|
| INV EXA1 | 500 | 32.2 | 6.4 |
| INV EXA2 | 500 | 44.2 | 6.2 |
| INV EXA3 | 500 | 58.0 | 6.4 |

### 24 h Ibuprofen release from films prepared according to Example A

A 5 x 5 cm x cm film containing Ibuprofen, prepared according to Example A is weighed and placed in ajar. 100 mL of Phosphate buffer pH 7.4 is added to the film and the jar is sealed and shaken at room temperature for 24 hours using a ThermoScientific MaxQ4000 orbital shaker. Samples of solutions resulted after 24 h are analyzed for Ibuprofen content by measuring the maximum absorption at λ = 264 nm using a Varian, Model "Cary 50 Tablet," UV-Visible Spectrophotometer from Agilent Technologies, USA. The calibration curve for the study was obtained by preparing standards of Ibuprofen of known concentrations in Phosphate Buffer pH 7.4. Table Ab shows the Ibuprofen released in 24 h from the films prepared according to Example A and the theoretical amount of Ibuprofen loaded based on Example A.

**Table Ab**

| Example | Dry film weight (g) | Theoretical IBU content | | Ibuprofen Released in 24 h | |
|---|---|---|---|---|---|
| | | mg | mg/g film | mg | mg/g film |
| INV EXA1 | 0.1467 | 20.33 | 138.7 | 18.12 | 123.5 |
| | 0.2904 | 40.27 | 138.7 | 37.23 | 128.2 |
| INV EXA2 | 0.1490 | 26.94 | 180.8 | 23.54 | 158.0 |
| | 0.2963 | 53.55 | 180.7 | 47.47 | 160.2 |
| INV EXA3 | 0.1507 | 33.87 | 224.7 | 31.43 | 208.6 |
| | 0.2877 | 64.66 | 224.7 | 60.75 | 211.2 |

Calculation of theoretical IBU content in the dry film is illustrated for INV EXA1 as follows: 500 g of Carbopol® 981 NF (pH 5.5)/TPU1 blend is obtained according to Example 1 by mixing 250 g 1% Carbopol® 981 NF (pH 5.5) and 250 g 3% TPU1 solution in THF.

According to INV EXA1 Table Aa, to this blend is added 32.2 g 5% Ibuprofen solution in THF. From this blend films are cast and dried which implies that upon drying all solvents are evaporated and the remaining dry film consists of Carbopol® 981 NF (pH 5.5)/TPU1/IBU that originate from each gel or solution used above to prepare the mixture used to draw down the films.
Amount of Carbopol® 981 NF (pH 5.5) in 250 g 1% dispersion: 250 x (1/100) = 2.5g
Amount of TPU1 in 250 g 3% THF solution: 250 x (3/100) = 7.5 g
Amount of IBU in 32.2 g 5% THF solution: 32.2 x (5/100) = 1.61 g
Total amount of solids in the dry film: 2.5 g Carbopol® 981 NF (pH 5.5) + 7.5 g
TPU1 + 1.61 g IBU = 11.61 g
% IBU in the dry film: (1.61g/11.61g) x 100 = 13.86%

Knowing the %IBU in the dry film one can estimate the theoretical amount of IBU in the films used for Ibuprofen release studies according to the following example:
Table Ab INV EXA1: dry film weight 0.1467 g
Amount of IBU in 0.1467 g is: 0.1467 x 13.86/100 = 0.02033 g = 20.33 mg
To calculate mg IBU/g dry film: 20.33 mg IBU/0.1467 g film = 138.6 mg/g dry film This algorithm can be applied to all examples listed in Table Ab taking in consideration the information provided in Table Aa for each inventive example.

### Example B - Preparation of Ibuprofen/Pemulen™ TR2 NF (pH 4.5)/TPU1 films and Ibuprofen release from the prepared films

To 500 g blend of Pemulen TR2™ and TPU1 prepared according to Example 7 add under continuous stirring 5% Ibuprofen solution in THF according to Table Ba. Let the resulted mixture stand overnight and measure viscosity and pH. From the mixture cast films using a 100 mil and 200 mil gap clearance bar, respectively, according to Example 2.

The films resulted from the compositions described herein will constitute INV EXB1, INV EXB2 and INV EXB3.

Table Ba illustrates the amount of Ibuprofen solution added, the pH and viscosity of the Ibuprofen containing blend that are used to cast INV EXB1, INV EXB2 and INV EXB3, respectively.

**Table Ba**

| Example | Amount of Pemulen TR2™ (pH 4.5)/TPU1 Blend (g) | Amount of 5% IBU in THF (g) | pH of resulted mixture | Viscosity of resulted mixture |
|---|---|---|---|---|
| INV EXB1 | 500 | 32.2 | 6.2 | 3,200 |
| INV EXB2 | 500 | 44.2 | 6.3 | 2,950 |
| INV EXB3 | 500 | 58.0 | 6.1 | 2,650 |

### 24 h Ibuprofen release for films prepared according to Example B

A 5 x 5 cm x cm films containing Ibuprofen, prepared according to Example B is weighed and placed in a jar. 100 mL of Phosphate buffer pH 7.4 is added to the film and the jar is sealed and shaken at room temperature for 24 hours using a ThermoScientific MaxQ4000 orbital shaker. Samples of solutions resulted after 24 h are analyzed for Ibuprofen content by measuring the maximum absorption at λ = 264 nm using a Varian, Model "Cary 50 Tablet," UV-Visible Spectrophotometer from Agilent Technologies, USA. The calibration curve for the study is obtained by preparing standards of Ibuprofen of known concentrations in Phosphate Buffer pH 7.4. Table Bb shows the Ibuprofen released in 24 h from the films prepared according to Example B and the theoretical amount of Ibuprofen loaded based on Example B.

**Table Bb**

| Example | Dry film weight (g) | Theoretical IBU content | | Ibuprofen Released in 24 h | |
|---|---|---|---|---|---|
| | | mg | mg/g film | mg | mg/g film |
| INV EXB1 | 0.1337 | 18.54 | 138.7 | 17.05 | 127.52 |
| | 0.2703 | 37.49 | 138.7 | 33.56 | 124.16 |
| INV EXB2 | 0.1443 | 26.10 | 180.8 | 26.72 | 185.16 |
| | 0.3005 | 54.33 | 180.8 | 53.70 | 178.70 |
| INV EXB3 | 0.1556 | 34.98 | 224.7 | 34.77 | 223.46 |
| | 0.2824 | 63.48 | 224.7 | 61.94 | 219.33 |

| | | | | | |
|---|---|---|---|---|---|
| *Theoretical IBU content in the dry film is calculated based on the same algorithm used in Example A. | | | | | |

### Comparative Example A 24 h Ibuprofen release from Ibuprofen-TPU commercial foam wound dressing

9.8 x 9.8 cm x cm commercial Biatain® Ibu (Coloplast A/S, Denmark) Ibuprofen containing TPU foam wound dressing is weighed and placed in a jar. 600 mL Phosphate buffer pH 7.4 is added to the foam and the jar is sealed and shaken at room temperature for 24 hours using a ThermoScientific MaxQ4000 orbital shaker. Samples of solution resulted after 24 h are analyzed for Ibuprofen content by measuring the maximum absorption at λ = 264 nm using a Varian, Model "Cary 50 Tablet," UV-Visible Spectrophotometer from Agilent Technologies, USA. The calibration curve for the study was obtained by preparing standards of Ibuprofen of known concentrations in Phosphate Buffer pH 7.4. Results are illustrated in Table CEXA

**Table CEXA**

| Example | Sample weight (g) | Ibuprofen released 24 h | |
|---|---|---|---|
| | | mg | mg/g foam |
| COMP EXA | 6.8141 | 315.57 | 46.31 |
| | 6.7988 | 315.30 | 46.37 |

The amount of Ibuprofen released in 24 h expressed as mg IBU/g foam was calculated by dividing the amount of Ibuprofen in mg obtained experimentally to the Sample weight used in the study: 315.57 mg IBU/6.8141 g foam = 46.31 mg IBU/g foam.

### Example C - Preparation of Metronidazole/Pemulen™ TR2 NF (pH 4.5)/TPU1 films and Metronidazole release from the prepared films

To 200 g 1% aqueous Pemulen™ TR2 neutralized with Tromethamine to pH 4.5 (Table Ca) add 1% aqueous Metronidazole (MTNZ) solution according to Table Cb. To the resulted MTNZ/ Pemulen™ TR2 mixture add 200 g 3% solution of TPU1 in THF : water 90 : 10. Let the final mixture stand at room temperature overnight and measure viscosity and pH. Table Cc shows the viscosity and pH of the MTNZ/Pemulen™ TR2 NF/TPU1 blends. From these blends cast films according to Example 2. The films resulted from the compositions described herein constitute INV EXC1, INV EXC2 and INVEXC3.

**Table Ca**

| Amount of Pemulen™ TR2 1% aqueous dispersion (g) | Amount of 30% aqueous Tromethamine solution used (g) | pH of partially neutralized Pemulen™ TR2 1% aqueous dispersion | Viscosity of partially neutralized Pemulen™ TR2 1% aqueous dispersion (cPs) |
|---|---|---|---|
| 200 | 2.2 | 4.76 | 20,000 |
| 200 | 2.2 | 4.80 | 23,300 |
| 200 | 2.2 | 4.92 | 22,500 |

**Table Cb**

| Amount of 1% aqueous MTNZ solution added to 200 g partially neutralized Pemulen™ TR2 according to Table d1 (g) | pH of MTNZ/partially neutralized Pemulen ™ TR2 resulted mixture | Viscosity of MTNZ/partially neutralized Pemulen™ TR2 resulted mixture (cPs) |
|---|---|---|
| 8.1 | 4.78 | 7,480 |
| 42.1 | 4.84 | 14,700 |
| 89 | 4.88 | 9,000 |

**Table Cc**

| | Amount of 3% SP80A150 THF/water 90/10 solution added to the MTNZ/PemulenTR2 blend prepared as per Table d2 | pH of MTNZ/PEMTR2/SP80A150 blends | Viscosity of MTNZ/PEMTR2/SP 80A150 blends |
|---|---|---|---|
| | (g) | | (cPs) |
| INV EXC1 | 200 | 6.23 | 4,500 |
| INV EXC2 | 200 | 6.42 | 4,800 |
| INV EXC3 | 200 | 6.45 | 4,900 |

### 24 h Metronidazole release from films prepared according to Example C

A 5 x 5 cm x cm film containing MTNZ, prepared according to Example d is weighed and placed in ajar. HCl 0.1M (aqueous) is added to the film according to Table Cd and the jar is sealed and shaken at room temperature for 24 hours using a ThermoScientific MaxQ4000 orbital shaker. Samples of solutions resulted after 24 h are analyzed for MTNZ content by measuring the maximum absorption at λ = 277 nm using a Varian, Model "Cary 50 Tablet," UV-Visible Spectrophotometer from Agilent Technologies, USA. The calibration curve for the study is obtained by preparing standards of MTNZ of known concentrations in HCl 0.1M. Table Cd shows the MTNZ released in 24 h from the films prepared according to Example C and the theoretical amount of MTNZ loaded based on Example C.

**Table Cd**

| Example | Volume of HCl 0.1M Used for the assay (mL) | Dry film weight (g) | Theoretical MTNZ content | | MTNZ Released in 24 h | |
|---|---|---|---|---|---|---|
| | | | mg | mg/g film | mg | mg/g film |
| INV EXC1 | 100 | 0.1337 | 1.23 | 9.2 | 1.32 | 9.8 |
| | 100 | 0.1282 | 1.18 | 9.2 | 1.26 | 9.8 |
| INV EXC2 | 200 | 0.1265 | 6.325 | 50 | 6.36 | 50.3 |
| | 200 | 0.1291 | 6.455 | 50 | 6.60 | 51.1 |
| INV EXC3 | 600 | 0.1108 | 11.08 | 100 | 11.07 | 99.9 |
| | 600 | 0.1221 | 12.21 | 100 | 12.29 | 100.65 |

### Example D - Preparation of Lidocaine/Pemulen™ TR2/TPU1 films and Lidocaine release from the prepared films

To 200 g 1% aqueous Pemulen™ TR2 neutralized with Tromethamine according to Table Da add 20% ethanolic solution of Lidocaine according to Table Db. To the resulted Lidocaine/ Pemulen™ TR2 mixture add 200 g 3% solution of TPU1 in THF : water 90 : 10. Let the final mixture stand at room temperature overnight and measure viscosity and pH. Table Dc shows the viscosity and pH of the Lidocaine/Pemulen™ TR2 NF/TPU1 blends. From these blends cast films according to Example 2. The films resulted from the compositions described herein constitute INV EXD1, INV EXD2, INV EXD3 and INV EXD4.

**Table Da**

| Sample | Amount of 30% aq Tromethamine added to 200 g 1% Pemulen TR2 | Viscosity of neutralized Pemulen™ TR2 | pH of neutralized Pemulen™ TR2 |
|---|---|---|---|
| | (g) | (cPs) | |
| 1 | 0 | 3,800 | 2.83 |
| 2 | 2.0 | 21,400 | 4.55 |
| 3 | 4.2 | 22,000 | 5.48 |
| 4 | 9.07 | not measured | 7.05 |

**Table Db**

| Sample | Amount of 20% Lidocaine in Ethanol added to 200 g neutralized Pemulen™ TR2 (g) | Viscosity of resulted Lidocaine/Pemulen™ TR2 (cPs) | pH of resulted Lidocaine/Pemulen™ TR2 |
|---|---|---|---|
| 1 | 2 | 10,700 | 3.87 |
| 2 | 2 | 22,200 | 4.99 |
| 3 | 2 | 25,500 | 5.77 |
| 4 | 2 | 17,000 | 7.39 |

**Table Dc**

| | pH of Lidocaine/Pemulen™ TR2/TPU1 blends | Viscosity of Lidocaine/Pemulen™ TR2/TPU1 blends |
|---|---|---|
| | | (cPs) |
| INV EXD1 | 5.84 | 4,340 |
| INV EXD2 | 6.38 | 5,100 |
| INV EXD3 | 6.75 | 4,800 |
| INV EXD4 | 7.83 | 4,600 |

### 24 h Lidocaine release from films prepared according to Example D

A 5 x 5 cm x cm film containing Lidocaine, prepared according to Example D is weighed and placed in ajar. HCl 0.1M (aqueous) is added to the film according to Table Dd and the jar is sealed and shaken at room temperature for 24 hours using a ThermoScientific MaxQ4000 orbital shaker. Samples of solutions resulted after 24 h are analyzed for Lidocaine content by measuring the maximum absorption at λ = 263 nm using a Varian, Model "Cary 50 Tablet," UV-Visible Spectrophotometer from Agilent Technologies, USA. The calibration curve for the study is obtained by preparing standards of Lidocaine of known concentrations in HCl 0.1 M. Table Dd shows the Lidocaine released in 24 h from the films prepared according to Example D and the theoretical amount of Lidocaine loaded based on Example D.

**Table Dd**

| Example | Volume of HCl 0.1M Used for the assay (mL) | Dry film weight (g) | Theoretical Lidocaine content | | Lidocaine Released in 24 h | |
|---|---|---|---|---|---|---|
| | | | Mg | mg/g film | mg | mg/g film |
| INV EXD1 | 50 | 0.1411 | 6.71 | 47.55 | 6.52 | 46.20 |
| | 50 | 0.1392 | 6.62 | 47.55 | 6.44 | 46.26 |
| INV EXD2 | 50 | 0.1395 | 6.64 | 47.60 | 6.48 | 46.45 |
| | 50 | 0.1356 | 6.45 | 47.56 | 6.37 | 46.97 |
| INV EXD3 | 50 | 0.1439 | 6.85 | 47.60 | 6.10 | 42.39 |
| | 50 | 0.1512 | 7.18 | 47.48 | 6.13 | 40.54 |
| INV EXD4 | 50 | 0.1662 | 7.91 | 47.59 | 5.71 | 34.35 |
| | 50 | 0.1782 | 8.48 | 47.58 | 6.22 | 34.90 |

### Example E - Preparation of Lidocaine HCl/Pemulen™ TR2/TPU1 films and Lidocaine release from the prepared films

To 200 g 1% aqueous Pemulen™ TR2 neutralized with Tromethamine according to Table Ea add 18.75% aqueous solution of Lidocaine Hydrochloride (LID HCl) according to Table Eb. To the resulted LID HCl/ Pemulen™ TR2 mixture add 200 g 3% solution of TPU1 in THF : water 90 : 10. Let the final mixture stand at room temperature overnight and measure viscosity and pH. Table Ec shows the viscosity and pH of the LID HCl/Pemulen™ TR2 NF/TPU1 blends. From these blends cast films according to Example 2. The films resulted from the compositions described herein constitute INV EXE1, INV EXE2 and INV EXE3.

**Table Ea**

| Sample | Amount of 30% aq Tromethamine added to 200 g 1% Pemulen™ TR2 | Viscosity of neutralized Pemulen™ TR2 | pH of neutralized Pemulen™ TR2 |
|---|---|---|---|
| | (g) | (cPs) | |
| 1 | 1.75 | 18,100 | 4.52 |
| 2 | 4.2 | 21,200 | 5.49 |
| 3 | 8.5 | 13,200 | 6.95 |

**Table Eb**

| Sample | Amount of 18.75% aqueous LID HCl added to 200 g neutralized Pemulen™ TR2 (g) | Viscosity of resulted LID HCl/Pemulen™ TR2 (cPs) | pH of resulted LID HCl/Pemulen™ TR2 |
|---|---|---|---|
| 1 | 2 | 18,300 | 4.45 |
| 2 | 2 | 27,300 | 5.48 |
| 3 | 2 | 15,300 | 6.93 |

**Table Ec**

| | pH of LID HCl/Pemulen™ TR2/TPU1 blends | Viscosity of LID HCl/Pemulen™ TR2/TPU1 blends (cPs) |
|---|---|---|
| INV EXE 1 | 5.86 | 3,300 |
| INV EXE2 | 6.44 | 3,700 |
| INV EXE3 | 7.26 | 3,400 |

### 24 h Lidocaine HCl release from films prepared according to Example E

A 5 x 5 cm x cm film containing Lidocaine HCl, prepared according to Example E is weighed and placed in a jar. 50 mL of Solution A (Na⁺/Ca²⁺ 142mmol/L//2.5 mmol/L - aqueous solution) is added to the film and the jar is sealed and shaken at room temperature for 24 hours using a ThermoScientific MaxQ4000 orbital shaker. Samples of solutions resulted after 24 h are analyzed for Lidocaine HCl content by measuring the maximum absorption at λ = 263 nm using a Varian, Model "Cary 50 Tablet," UV-Visible Spectrophotometer from Agilent Technologies, USA. The calibration curve for the study is obtained by preparing standards of Lidocaine HCl of known concentrations solution A, respectively. Table Ed shows the 24 h Lidocaine HCl released in Solution A from the films prepared according to Example E and the theoretical amount of Lidocaine HCl loaded based on Example E.

**Table Ed**

| Example | Dry film weight (g) | Theoretical Lidocaine content | | Lidocaine Released in 24 h | |
|---|---|---|---|---|---|
| | | mg | mg/g film | mg | mg/g film |
| INV EXE1 | 0.1419 | 6.35 | 44.75 | 6.01 | 42.35 |
| | 0.1567 | 7.02 | 44.79 | 6.50 | 41.48 |
| INV EXE2 | 0.1506 | 6.74 | 44.75 | 5.95 | 39.50 |
| | 0.1642 | 7.35 | 44.76 | 6.45 | 39.28 |
| INV EXE3 | 0.1591 | 7.12 | 44.75 | 5.39 | 33.88 |
| | 0.1827 | 8.18 | 44.77 | 6.26 | 34.26 |

## Claims

1. A film, comprising:
a) a partially-neutralized, cross-linked poly(acrylic) acid polymer; and
b) a hydrophilic thermoplastic polyurethane;
wherein, upon mixing, (a) and (b) form an interpenetrating network resulting in a homogenous film.

2. The film of claim 1, wherein the cross-linked poly(acrylic) acid polymer is a carbomer copolymer, a carbomer homopolymer, carbomer interpolymer, or a polycarbophil.

3. The film of any claim 1 or 2, wherein the poly(acrylic) acid polymer is cross-linked with an allyl ether cross-linking agent, preferably wherein the allyl ether cross-linking agent comprises one or more of allyl pentaerythritol, allyl sucrose, trimethpropanediolyl ether (TMPDE) and divinyl glycol.

4. The film of any of the preceding claims, wherein the thermoplastic polyurethane comprises the reaction product of (i) a polyisocyanate component comprising at least one aliphatic diisocyanate; (ii) a polyol component comprising at least one polyether polyol; and (iii) a chain extender component.

5. The film of claim 4, wherein the chain extender comprises an aliphatic diol.

6. The film of claim 5, wherein the polyol component comprises at least one polyethylene glycol having a number average molecular weight (Mn) of at least 300, or wherein the polyol component comprises at least one polyethylene glycol having number average molecular weight (Mn) of at least 1450, or wherein the polyol component comprises a blend of polyethylene glycol having number average molecular weights (Mn) of at least 1450 and at least 8000.

7. The film of any of the preceeding claims in which the cross-linked poly(acrylic) acid polymer is partially neutralized.

8. The film of any of the preceding claims, wherein water absorption of the film is from 400% to 3000% by weight of dry film.

9. The film of any of the preceding claims, wherein the ratio of (a) to (b) is from 1:1 to 1:60.

10. The film of any of the preceding claims, wherein the hydrophilic thermoplastic polyurethane forms 97 to 50 wt% of the total weight of the composition.

11. The film of any of the preceding claims, further comprising a therapeutically active agent dispersed therein.

12. The film of any of the preceding claims, the film having, upon drying:
(a) a tensile strength of from 5 MPa to 50 MPa;
(b) a percent elongation from 100% to 700%; and
(c) a Young's modulus from 3 MPa to 150 MPa.

13. The film of any of the preceding claims, the film having, upon drying, a Moisture Vapor Transmission Rate (MVTR) of from 1000 to 8000 g/(m²xday).

14. A wound dressing comprising the film of any of claims 1 to 13.

15. The wound dressing of claim 14, wherein the film comprises a single layer, preferably wherein the thickness of the single layer is from 12.7 µm to 2.54 mm (from 0.5 mil to 100 mil).

16. The wound dressing of claim 14 or 15, further comprising a backing and a facing.

17. A patch comprising the film of any of claims 1 to 13.

18. The patch according to claim 17, wherein the patch further comprises one or more of a pharmaceutical, a biologically active compound, an absorptive material, a personal care compound, an active ingredient, a therapeutic aid, or combinations thereof.

19. The patch of claim 17 or 18, wherein the patch is an adhesive patch or a non-adhesive patch.

## Patentansprüche

1. Film, umfassend:
a) ein partiell neutralisiertes vernetztes Polyacrylsäure-Polymer; und
b) ein hydrophiles thermoplastisches Polyurethan;
wobei (a) und (b) beim Mischen ein interpenetrierendes Netzwerk bilden, das zu einem homogenen Film führt.

2. Film gemäß Anspruch 1, wobei das vernetzte Polyacrylsäure-Polymer ein Carbomer-Copolymer, ein Carbomer-Homopolymer, ein Carbomer-Interpolymer oder ein Polycarbophil ist.

3. Film gemäß einem der Ansprüche 1 oder 2, wobei das Polyacrylsäure-Polymer mit einem Allylether-Vernetzungsmittel vernetzt ist, wobei vorzugsweise das Allylether-Vernetzungsmittel eines oder mehrere aus Allylpentaerythrit, Allylsaccharose, Trimethylpropandiolylether (TMPDE) und Divinylglycol umfasst.

4. Film gemäß einem der vorstehenden Ansprüche, wobei das thermoplastische Polyurethan das Reaktionsprodukt von (i) einer Polyisocyanatkomponente, die wenigstens ein aliphatisches Diisocyanat umfasst, (ii) einer Polyolkomponente, die wenigstens ein Polyetherpolyol umfasst, und (iii) eine Kettenverlängererkomponente umfasst.

5. Film gemäß Anspruch 4, wobei der Kettenverlängerer ein aliphatisches Diol umfasst.

6. Film gemäß Anspruch 5, wobei die Polyolkomponente wenigstens ein Polyethylenglycol mit einem Zahlenmittel des Molekulargewichts (Mn) von wenigstens 300 umfasst oder wobei die Polyolkomponente wenigstens ein Polyethylenglycol mit einem Zahlenmittel des Molekulargewichts (Mn) von wenigstens 1450 umfasst oder wobei die Polyolkomponente ein Gemisch von Polyethylenglycolen mit Zahlenmitteln des Molekulargewichts (Mn) von wenigstens 1450 und höchstens 8000 umfasst.

7. Film gemäß einem der vorstehenden Ansprüche, wobei das vernetzte Polyacrylsäure-Polymer partiell neutralisiert ist.

8. Film gemäß einem der vorstehenden Ansprüche, wobei die Wasserabsorption des Films 400 bis 3000 Gew.-% des trockenen Films beträgt.

9. Film gemäß einem der vorstehenden Ansprüche, wobei das Verhältnis von (a) zu (b) 1:1 bis 1:60 beträgt.

10. Film gemäß einem der vorstehenden Ansprüche, wobei das hydrophile thermoplastische Polyurethan 97 bis 50 Gew.-% des Gesamtgewichts der Zusammensetzung bildet.

11. Film gemäß einem der vorstehenden Ansprüche, weiterhin umfassend einen darin dispergierten therapeutischen Wirkstoff.

12. Film gemäß einem der vorstehenden Ansprüche, wobei der Film nach dem Trocknen Folgendes aufweist:
(a) eine Zugfestigkeit von 5 MPa bis 50 MPa;
(b) eine prozentuale Dehnung von 100% bis 700%; und
(c) einen Youngschen Modul von 3 MPa bis 150 MPa.

13. Film gemäß einem der vorstehenden Ansprüche, wobei der Film nach dem Trocknen eine Wasserdampfdurchlässigkeit von 1000 bis 8000 g/(m²·Tag) aufweist.

14. Wundverband, der den Film gemäß einem der Ansprüche 1 bis 13 umfasst.

15. Wundverband gemäß Anspruch 14, wobei der Film eine einzige Schicht umfasst, wobei vorzugsweise die Dicke der einzigen Schicht 12,7 µm bis 2,54 mm (0,5 mil bis 100 mil) beträgt.

16. Wundverband gemäß Anspruch 14 oder 15, weiterhin umfassend eine Trägerschicht und eine Deckschicht.

17. Pflaster, das den Film gemäß einem der Ansprüche 1 bis 13 umfasst.

18. Pflaster gemäß Anspruch 17, wobei das Pflaster weiterhin eines oder mehrere aus einem Arzneimittel, einer biologisch aktiven Verbindung, einem saugfähigen Material, einer Körperpflegeverbindung, einem Wirkstoff, einem therapeutischen Hilfsmittel oder Kombinationen davon umfasst.

19. Pflaster gemäß Anspruch 17 oder 18, wobei das Pflaster ein Klebepflaster oder ein nichtklebendes Pflaster ist.

## Revendications

1. Film, comprenant :
(a) un polymère d'acide poly(acrylique) réticulé partiellement neutralisé ; et
(b) un polyuréthane thermoplastique hydrophile ;
dans lequel, lorsqu'ils sont en mélange, (a) et (b) forment un réseau interpénétrant résultant en un film homogène.

2. Film selon la revendication 1, dans lequel le polymère d'acide poly(acrylique) réticulé est un co-polymère de carbomère, un homo-polymère de carbomère, un inter-polymère de carbomère, ou un polycarbophile.

3. Film selon l'une quelconque des revendications 1 ou 2, dans lequel le polymère d'acide poly(acrylique) est réticulé avec un agent de réticulation d'éther d'allyle, de préférence dans lequel l'agent de réticulation d'éther d'allyle comprend un ou plusieurs de pentaérythritol d'allyle, de sucrose d'allyle, d'éther de triméthpropanédiolyl (TMPDE) et de glycol de divinyle.

4. Film selon l'une quelconque des revendications précédentes, dans lequel le polyuréthane thermoplastique comprend le produit de réaction (i) d'un composant polyisocyanate comprenant au moins un disocyanate aliphatique ; (ii) un composant polyol comprenant au moins un polyol de polyéther ; et (iii) un composant allongeur de chaîne.

5. Film selon la revendication 4, dans lequel l'allongeur de chaîne comprend un diol aliphatique.

6. Film selon la revendication 5, dans lequel le composant polyol comprend au moins un polyéthylène glycol présentant une masse moléculaire moyenne en nombre (Mn) d'au moins 300, ou dans lequel le composant polyol comprend au moins un polyéthylène glycol présentant une masse moléculaire moyenne en nombre (Mn) d'au moins 1450, ou dans lequel le composant polyol comprend un mélange de polyéthylène glycol présentant des masses moléculaires moyennes en nombre (Mn) d'au moins 1450 et au moins 8000.

7. Film selon l'une quelconque des revendications précédentes dans lequel le polymère d'acide poly(acrylique) réticulé est partiellement neutralisé.

8. Film selon l'une quelconque des revendications précédentes, dans lequel l'absorption d'eau du film est de 400 % à 3000 % en poids de film sec.

9. Film selon l'une quelconque des revendications précédentes, dans lequel le rapport de (a) sur (b) est de 1:1 à 1:60.

10. Film selon l'une quelconque des revendications précédentes, dans lequel le polyuréthane thermoplastique hydrophile forme 97 à 50 % en poids du poids totale de la composition.

11. Film selon l'une quelconque des revendications précédentes, comprenant en outre un agent thérapeutiquement actif dispersé dans celui-ci.

12. Film selon l'une quelconque des revendications précédentes, le film présentant, lors d'un séchage :
(a) une résistance à la traction de 5 MPa à 50 MPa ;
(b) un pourcentage d'allongement de 100 % à 700 % ; et
(c) un module de Young de 3 MPa à 150 MPa.

13. Film selon l'une quelconque des revendications précédentes, le film présentant, lors d'un séchage, un taux de transmission de vapeur humide (MVTR) de 1000 à 8000 g/(m²xjour).

14. Pansement de plaie comprenant le film selon l'une quelconque des revendications 1 à 13.

15. Pansement de plaie selon la revendication 14, dans lequel le film comprend une couche unique, de préférence dans lequel l'épaisseur de la couche unique est de 12,7 µm à 2,54 mm (de 0,5 mil à 100 mil).

16. Pansement de plaie selon la revendication 14 ou 15, comprenant en outre un côté arrière et un côté avant.

17. Patch comprenant le film selon l'une quelconque des revendications 1 à 13.

18. Patch selon la revendication 17, dans lequel le patch comprend en outre un ou plusieurs d'un produit pharmaceutique, un composé biologiquement actif, un matériau absorbant, un composé de soin personnel, un ingrédient actif, une aide thérapeutique, ou des combinaisons de ceux-ci.

19. Patch selon la revendication 17 ou 18, dans lequel le patch est un patch adhésif ou un patch non-adhésif.
